# EUROPEAN PATENT SPECIFICATION

(11) **EP 3 090 065 B1**
(45) Date of publication and mention of the grant of the patent: **11.12.2019**
(21) Application number: 14876692.6
(22) Date of filing: 30.12.2014
(51) Int. Cl.: C12Q 1/68

(54) **METHODS FOR MEASURING BIOMARKERS IN GASTROINTESTINAL CANCER**
VERFAHREN ZUR MESSUNG VON BIOMARKERN IN GASTROINTESTINALEM KREBS
PROCÉDÉS DE MESURE DE BIOMARQUEURS DANS UN CANCER GASTROINTESTINAL

(30) Priority: 30.12.2013 SG 201309689
(43) Date of publication of application: 09.11.2016
(73) Proprietor: Agency for Science, Technology and Research, Singapore 138632 (SG)
(72) Inventor: TAN, Boon Ooi Patrick, Singapore 138672 (SG); MURATANI, Masafumi, Ibaraki 305-8575 (JP); QAMRA, Aditi, Singapore 138672 (SG)
(74) Representative: Danner, Stefan
(86) International application number: PCT/SG2014/000625
(87) International publication number: WO 2015/102536

(56) References cited:
- EP-A1- 2 390 371
- WO-A2-2008/083251
- WO-A2-2008/103761
- TERRINONI A ET AL: "Cyclin D1 gene contains a cryptic promoter that is functional in human cancer cells", GENES, CHROMOSOMES & CANCER, XX, XX, vol. 31, 1 January 2001 (2001-01-01), pages 209-220, XP002527209, DOI: 10.1002/GCC.1137
- AXEL VISEL ET AL: "ChIP-seq accurately predicts tissue-specific activity of enhancers", NATURE, vol. 457, no. 7231, 12 February 2009 (2009-02-12), pages 854-858, XP055308081, ISSN: 0028-0836, DOI: 10.1038/nature07730
- A. BOULAY ET AL: "Transcription Regulation and Protein Subcellular Localization of the Truncated Basic Hair Keratin hHb1-?N in Human Breast Cancer Cells", JOURNAL OF BIOLOGICAL CHEMISTRY, vol. 276, no. 25, 13 April 2001 (2001-04-13), pages 22954-22964, XP055384050, US ISSN: 0021-9258, DOI: 10.1074/jbc.M101687200
- B. AKHTAR-ZAIDI ET AL: "Epigenomic Enhancer Profiling Defines a Signature of Colon Cancer", SCIENCE, vol. 336, no. 6082, 12 April 2012 (2012-04-12), pages 736-739, XP055384810, ISSN: 0036-8075, DOI: 10.1126/science.1217277
- NG JIA-HUI ET AL: "In Vivo Epigenomic Profiling of Germ Cells Reveals Germ Cell Molecular Signatures", DEVELOPMENTAL CELL, CELL PRESS, US, vol. 24, no. 3, 24 January 2013 (2013-01-24), pages 324-333, XP028976901, ISSN: 1534-5807, DOI: 10.1016/J.DEVCEL.2012.12.011
- LI ZHANG ET AL: "Genome-wide analysis of histone H3 lysine 27 trimethylation by ChIP-chip in gastric cancer patients", JOURNAL OF GASTROENTEROLOGY, SPRINGER-VERLAG, TO, vol. 44, no. 4, 10 March 2009 (2009-03-10) , pages 305-312, XP019719559, ISSN: 1435-5922
- ALI MORTAZAVI ET AL: "Mapping and quantifying mammalian transcriptomes by RNA-Seq", NATURE MET,, vol. 5, no. 7, 1 July 2008 (2008-07-01), pages 621-628, XP008148418, ISSN: 1548-7091, DOI: 10.1038/NMETH.1226
- GAL-YAM, E. N. ET AL.: 'Frequent switching of Polycomb repressive marks and DNA hypermethylation in the PC3 prostate cancer cell line' PROCEEDINGS OF THE NATIONAL ACADEMY OF SCIENCES vol. 105, no. 35, 02 September 2008, USA, pages 12979 - 12984, XP055355503
- MURATANI, M. ET AL.: 'Nanoscale chromatin profiling of gastric adenocarcinoma reveals cancer-associated cryptic promoters and somatically acquired regulatory elements' NATURE COMMUNICATIONS vol. 5, 10 July 2014, pages 1 - 14, XP055355507
- KWON OH-HYUNG ET AL: "Aberrant up-regulation of LAMB3 and LAMC2 by promoter demethylation in gastric cancer.", BIOCHEMICAL AND BIOPHYSICAL RESEARCH COMMUNICATIONS 25 MAR 2011, vol. 406, no. 4, 25 March 2011 (2011-03-25), pages 539-545, ISSN: 1090-2104
- Jiyeon Yun ET AL: "Gene silencing of EREG mediated by DNA methylation and histone modification in human gastric cancers", Laboratory Investigation, vol. 92, no. 7, 1 July 2012 (2012-07-01), pages 1033-1044, XP055109913, ISSN: 0023-6837, DOI: 10.1038/labinvest.2012.61
- Alvaro Rada-Iglesias ET AL: "A unique chromatin signature uncovers early developmental enhancers in humans", Nature, vol. 470, no. 7333, 15 December 2010 (2010-12-15), pages 279-283, XP055308006, GB ISSN: 0028-0836, DOI: 10.1038/nature09692
- Jason Ernst ET AL: "Mapping and analysis of chromatin state dynamics in nine human cell types", Nature, vol. 473, no. 7345, 23 March 2011 (2011-03-23), pages 43-49, XP055311128, GB ISSN: 0028-0836, DOI: 10.1038/nature09906

## Description

### FIELD OF THE INVENTION

The present invention relates to the field of molecular biology. In particular, the present invention relates to methods for determining the activity of a promoter.

### BACKGROUND OF THE INVENTION

Gastric cancer (GC) is a major cause of global cancer mortality. Most GCs are adenocarcinomas, and recent exome and whole-genome studies have revealed new GC driver genes and mutational signatures. Besides protein-coding genes, regulatory elements in non-coding genomic regions are also likely contributors to malignancy, as these elements can profoundly influence chromatin structure and gene expression. Few studies have explored the repertoire of regulatory elements somatically altered during gastric carcinogenesis, on a genomic scale.

Regulatory elements including promoters and enhancers can be identified as regions exhibiting histone modifications ("chromatin marks"). Exemplary analyses of histone modifications and their association with gastric cancer are described in Zhang et al. (Zhang, L. et al. (2009) J. Gastroenterol. 44, 305-312), Mortazavi et al. (Mortazavi, A. et al. (2008) Nature Meth. 5, 621-628), Kwon et al. (Kwon, O.H. et al. (2011) Biochem. Biophys. Res. Commun. 406, 539-545), Yun et al. (Yun, J. et al. (2012) Lab. Investigation 92, 1033-1044), Rada-Iglesias et al. (Rada-Iglesias, A. et al. (2011) Nature 470, 279-283), and Ernst et al. (Ernst, J. et al. (2011) Nature 473, 43-49). To date, most chromatin mark studies in cancer have used immortalized cell lines, since existing protocols require significant amounts of biological material. However, cancer lines cultured *in vitro* can display epigenetic patterns distinct from primary tumors, and cell lines may also undergo *in vitro* adaption, acquiring genetic and epigenetic changes due to extensive passaging. Identification of somatically acquired alterations using cancer cell lines is also difficult, as they often lack matched normal counterparts.

There is therefore a need to provide a method to measure chromatin marks that overcomes, or at least ameliorates, one or more of the disadvantages described above.

### SUMMARY OF THE INVENTION

The present invention provides a method, as specified in claim 1, for determining the presence or activity of at least one promoter in a cancerous biological sample relative to a non-cancerous biological sample, comprising; mapping an isolated nucleic acid comprising at least one promoter sequence obtained from said cancerous biological sample against a reference nucleic acid obtained from said non-cancerous biological sample to obtain a read per kilo-base per million (RPKM) value or fragments per kilo-base per million (FPKM) value for said at least one promoter; and determining the differential activity of the at least one promoter sequence in the nucleic acid relative to the activity of the at least one promoter in the reference nucleic acid sequence using said RPKM or FPKM value, wherein the step of determining the differential activity of the at least one promoter sequence comprises determining that the RKPM or FPKM value for the at least one promoter in the nucleic acid obtained from the cancerous biological sample is: (i) greater than between a 1 to 20-fold, such as a 1-fold, 2-fold, 3-fold, 4-fold or 5-fold, change in mean RPKM or FPKM value relative to the RPKM or FPKM value of the at least one promoter in the reference nucleic acid obtained from the non-cancerous biological sample; and (ii) greater than a 0.1 RPKM or FPKM range relative to the RPKM or FPKM value of the at least one promoter in the reference nucleic acid obtained from the non-cancerous biological sample; wherein the cancer is gastric cancer; and wherein the nucleic acid is isolated from said cancerous biological sample by immunoprecipitation of chromatin, wherein said nucleic acid comprises said at least one promoter, the immunoprecipitation being achieved by an antibody specific for a modified histone protein comprising at least one histone modification selected from the group consisting of H3K4me3, H3K4me1, and H3K27ac.

In one embodiment, the method further comprises: generating a matrix of sequencing tag counts for said at least one promoter based on the mapping step; and analyzing said matrix of sequencing tag counts prior to determining the differential activity the at least one promoter sequence in the nucleic acid.

### DEFINITIONS

The term "antigen binding protein" as used herein refers to antibodies, antibody fragments and other protein constructs, such as domains, which are capable of binding to an antigen.

The term "antibody" is used herein in the broadest sense to refer to molecules with an immunoglobulin-like domain and includes monoclonal, recombinant, polyclonal, chimeric, humanized, bispecific and hetero-conjugate antibodies; a single variable domain, a domain antibody, antigen binding fragments, immunologically effective fragments, single chain Fv, diabodies, Tandabs™, etc (for a summary of alternative "antibody" formats see Holliger and Hudson, Nature Biotechnology, 2005, Vol 23, No. 9, 1126-1136).

The phrase "single variable domain" refers to an antigen binding protein variable domain (for example, V_{H}, V_{HH}, V_{L}) that specifically binds an antigen or epitope independently of a different variable region or domain.

A "domain antibody" or "dAb" may be considered the same as a "single variable domain" which is capable of binding to an antigen. A single variable domain may be a human antibody variable domain, but also includes single antibody variable domains from other species such as rodent (for example, as disclosed in WO 00/29004), nurse shark and *Camelid* V_{HH} dAbs. Camelid V_{HH} are immunoglobulin single variable domain polypeptides that are derived from species including camel, llama, alpaca, dromedary, and guanaco, which produce heavy chain antibodies naturally devoid of light chains. Such V_{HH} domains may be humanized according to standard techniques available in the art, and such domains are considered to be "domain antibodies". As used herein V_{H} includes camelid V_{HH} domains.

As used herein the term "domain" refers to a folded protein structure which has tertiary structure independent of the rest of the protein. Generally, domains are responsible for discrete functional properties of proteins, and in many cases may be added, removed or transferred to other proteins without loss of function of the remainder of the protein and/or of the domain.

A "single variable domain" is a folded polypeptide domain comprising sequences characteristic of antibody variable domains. It therefore includes complete antibody variable domains and modified variable domains, for example, in which one or more loops have been replaced by sequences which are not characteristic of antibody variable domains, or antibody variable domains which have been truncated or comprise N- or C-terminal extensions, as well as folded fragments of variable domains which retain at least the binding activity and specificity of the full-length domain. A domain can bind an antigen or epitope independently of a different variable region or domain.

An antigen binding fragment may be provided by means of arrangement of one or more CDRs on non-antibody protein scaffolds such as a domain. The domain may be a domain antibody or may be a domain which is a derivative of a scaffold selected from the group consisting of CTLA-4, lipocalin, SpA, an Affibody, an avimer, GroEI, transferrin, GroES and fibronectin/adnectin, which has been subjected to protein engineering in order to obtain binding to an antigen.

An antigen binding fragment or an immunologically effective fragment may comprise partial heavy or light chain variable sequences. Fragments are at least 5, 6, 8 or 10 amino acids in length. Alternatively, the fragments are at least 15, at least 20, at least 50, at least 75, or at least 100 amino acids in length.

The term "specifically binds" as used throughout the present specification in relation to antigen binding proteins means that the antigen binding protein binds to an antigen with no or insignificant binding to other (for example, unrelated) proteins. The term however does not exclude the fact that the antigen binding proteins may also be cross-reactive with closely related molecules.

The terms "biological material" or "biological sample" as used herein refers to any material or sample, which includes an analyte as defined herein. Such samples may, for example, include samples derived from or comprising stool, whole blood, serum, plasma, tears, saliva, nasal fluid, sputum, ear fluid, genital fluid, breast fluid, milk, colostrum, placental fluid, amniotic fluid, perspirate, synovial fluid, ascites fluid, cerebrospinal fluid, bile, gastric fluid, aqueous humor, vitreous humor, gastrointestinal fluid, exudate, transudate, pleural fluid, pericardial fluid, semen, upper airway fluid, peritoneal fluid, fluid harvested from a site of an immune response, fluid harvested from a pooled collection site, bronchial lavage, urine, biopsy material, for example from all suitable organs, for example the lung, the muscle, brain, liver, skin, pancreas, stomach and the like, a nucleated cell sample, a fluid associated with a mucosal surface, hair, or skin.

The term "RPKM" as used herein, refers to Reads Per Kilobase per Million reads mapped. The term "FPKM" as used herein refers to Fragments Per Kilobase per Million fragments mapped. RPKM and FPKM are units to quantify abundance of any genomic feature, such as an exon, transcript or any genomic coordinates, determined by the abundance of sequencing reads aligning to it. The RPKM and FPKM measures normalize the abundance by relative length of the genomic unit as well as the total number of reads mapping to it, to facilitate transparent comparison of abundance levels within and between samples.

The term "matrix of sequencing tag counts" as used herein refers to a matrix of integer values of mapped "sequencing tags". The matrix may be in the form of a table with a row and column, wherein the value in the row (genomic region) and the column (tissue sample) of the matrix may indicate how many reads have been mapped to a genomic region, such as a promoter region or a histone modification region, for example the H3K4me3 region. Analogously, the rows of the matrix may also correspond to binding regions (with ChIP-Seq). The aforementioned "sequencing tags" as used herein refer to short DNA fragments isolated from samples which are mapped to a reference genome using an alignment tool (as mentioned in methods disclosed herein).

The term "bedtools" as used herein and in the context of the working examples refers to a set of published tools that are well known in the art for genomic analysis. For example, the "bedtools" may be used for the comparison, manipulation and annotation of genomic features in Browser Extensible Data (BED) and General Feature Format (GFF) format. Bedtools also supports the comparison of sequence alignments in BAM format to both BED and GFF features. The bedtools are extremely efficient and allow the user to compare large datasets (e.g. next-generation sequencing data) with both public and custom genome annotation tracks. For example, the "bedtools" may refer to "BEDTools", whereby the BEDTools can be combined with one another as well as with standard UNIX commands, thus facilitating routine genomics tasks as well as pipelines that can quickly answer intricate questions of large genomic datasets. In particular, such "bedtools" can be found at

### http://bioinformatics.oxfordjournals.org/content/26/6/841.full.

The term "obtained" or "derived from" as used herein is meant to be used inclusively. That is, it is intended to encompass any nucleotide sequence directly isolated from a biological sample or any nucleotide sequence derived from the sample.

The invention has been described generically herein. Each of the narrower species and sub-generic groupings falling within the generic disclosure also form part of the invention..

Particular embodiments are within the following claims and examples. In addition, where features or aspects of the invention are described in terms of Markush groups, those skilled in the art will recognize that the invention is also thereby described in terms of any individual member or subgroup of members of the Markush group.

### BRIEF DESCRIPTION OF THE DRAWINGS

The invention will be better understood with reference to the detailed description when considered in conjunction with the examples and the accompanying drawings, in which:

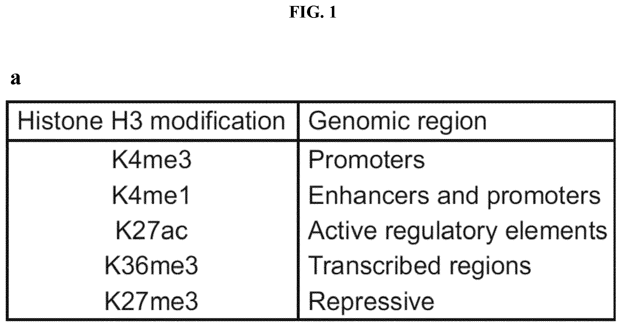

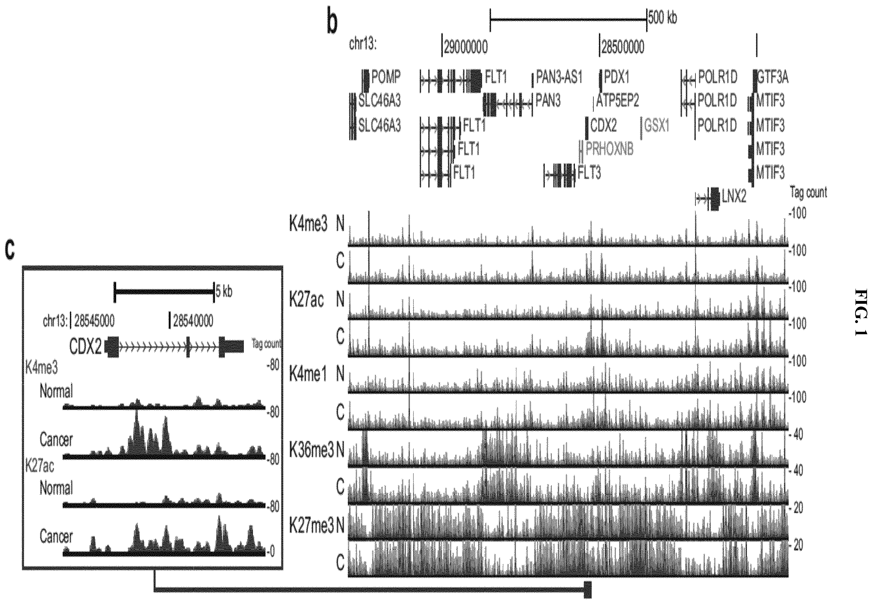

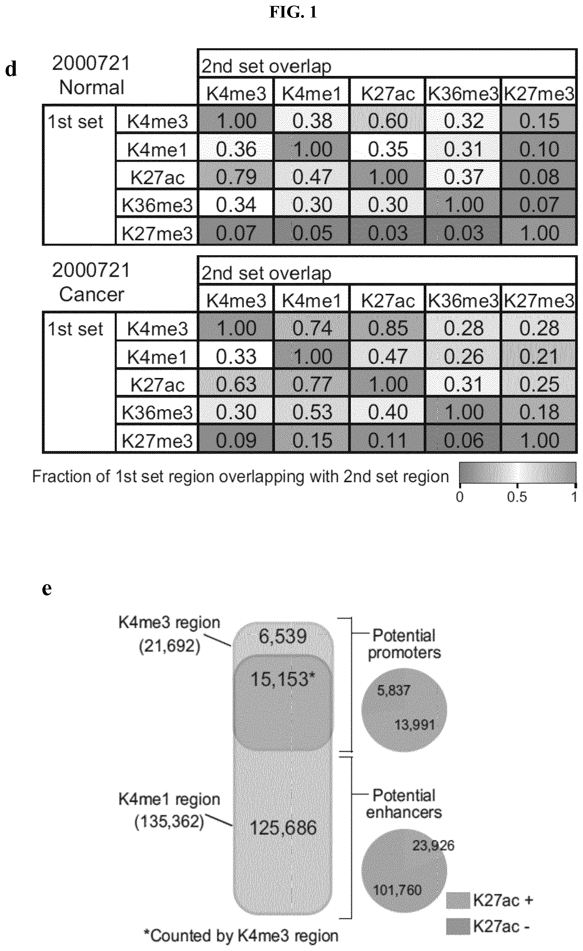

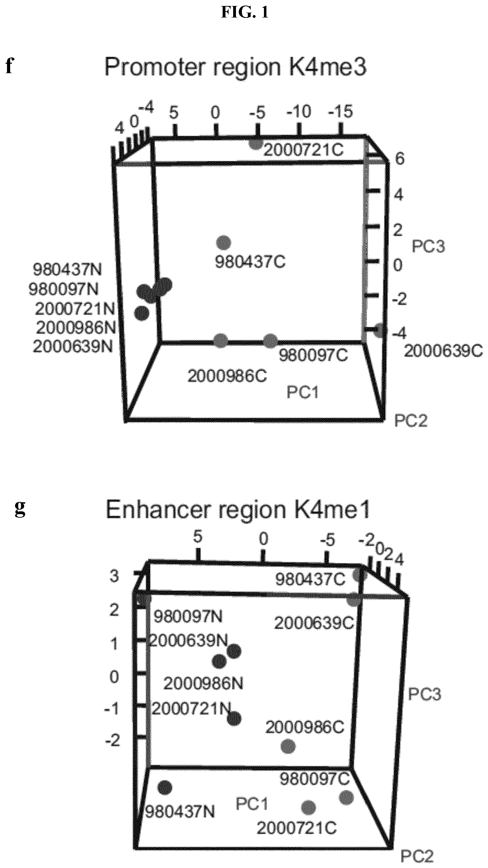

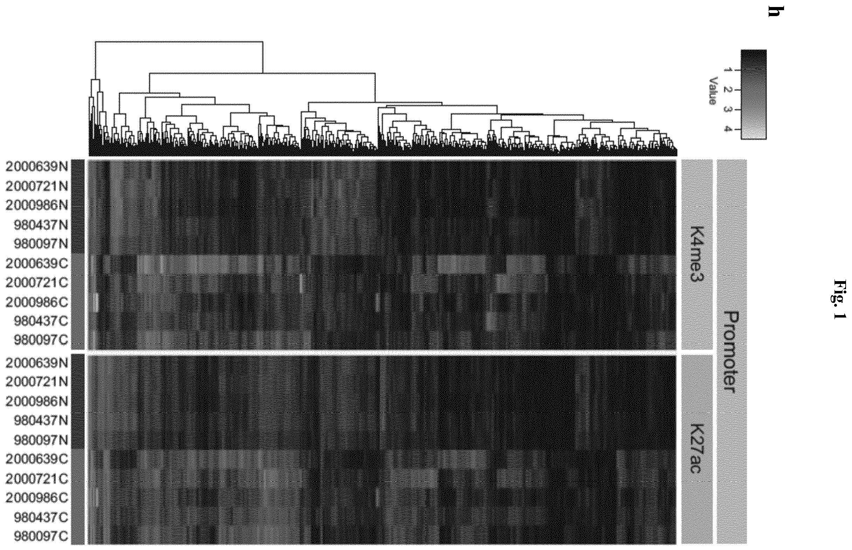

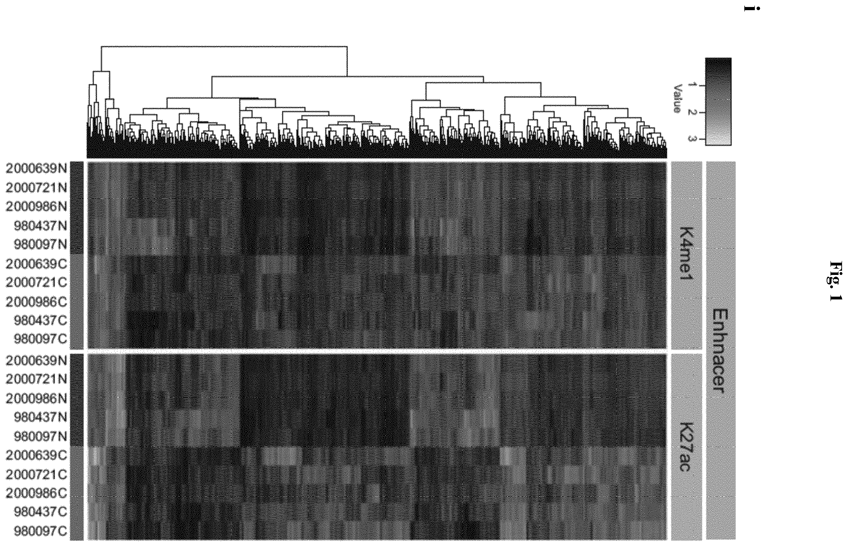

**Figure 1****. Nano-ChIPseq Chromatin Profiles of Primary Gastric Adenocarcinomas.** (a) Chromatin marks analyzed. (b) Chromatin profile of GC 2000721 and matched normal gastric tissue (C and N respectively). Shown is a representative UCSC Genome Browser display of the *CDX2* gene locus and adjacent genes. RefSeq transcripts and histone modifications are displayed. (c) Close-up view of the *CDX2* gene, demonstrating H3K4me3 and H3K27ac gain in GC. (d) Chromatin mark peak overlaps in normal (top) and GC (bottom). GC 2000721 is depicted. Numbers represent the fraction of 1st set regions (vertical) overlapping with 2nd set regions (horizontal). White = exclusive, darker gray = overlapping. (e) GC enhancers and promoters. Promoter and enhancer regions were further classified by their overlap with H3K27ac signals (right circles). (f, g) Principal Components Analysis (PCA) plots of normal (blue) and GC (red) samples based on somatically altered (f) promoter (H3K4me3) and (g) enhancer (H3K4me1) regions. The top 3 principal components were used. (h, i) Clustering heatmaps of normal (blue) and GC (red) samples based on somatically altered (h) promoter and (i) enhancer (H3K4me1) regions. H3K27ac patterns are also shown. Color intensities correspond to normalized RPKM values.

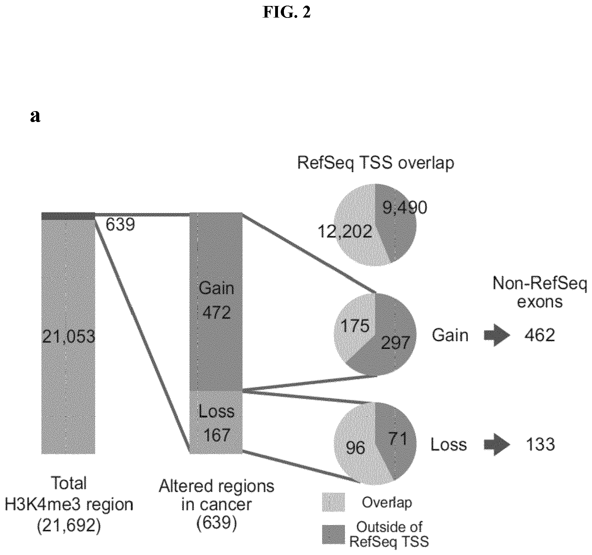

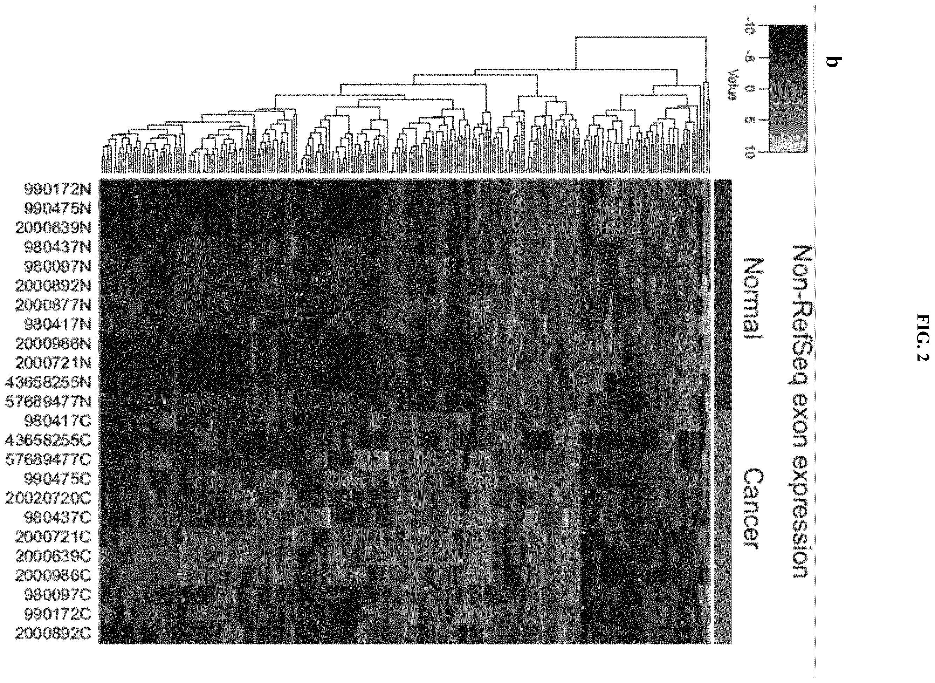

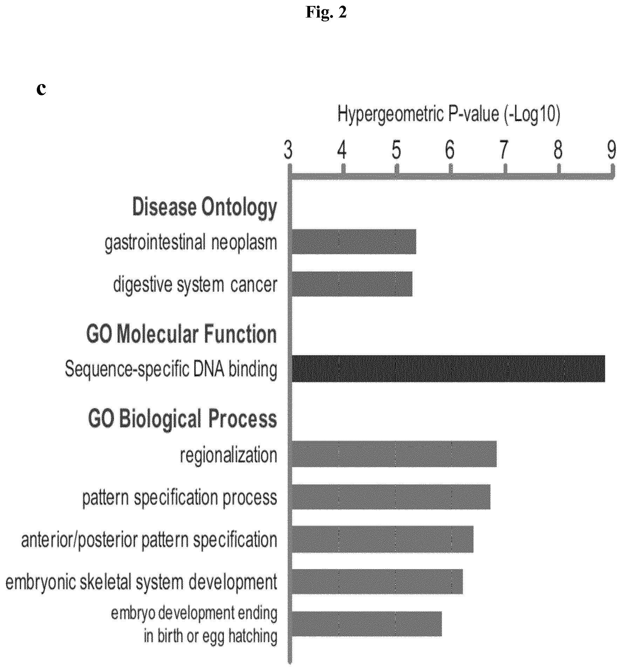

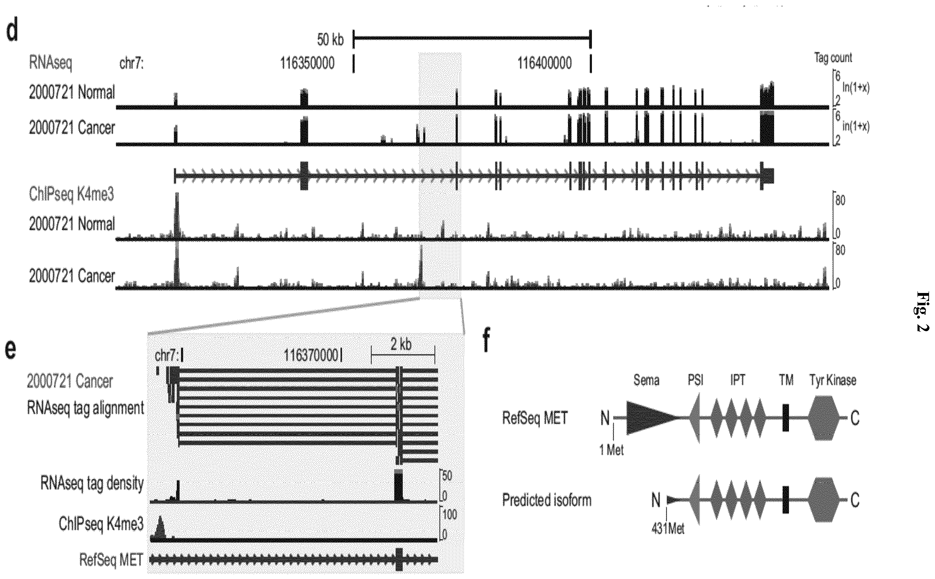

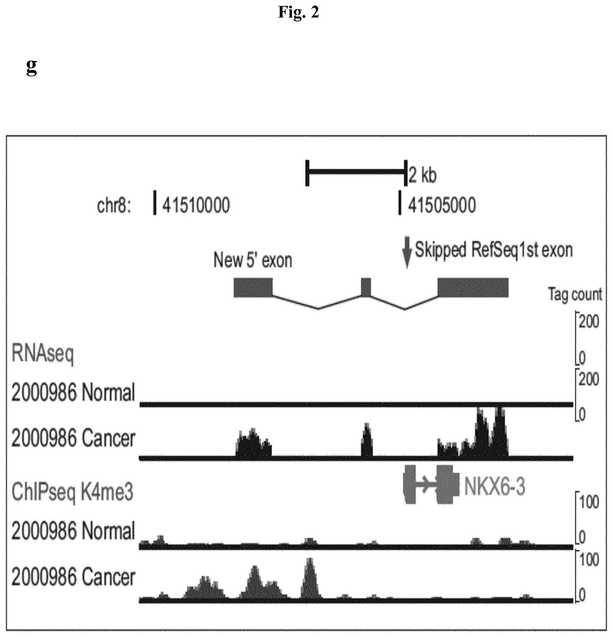

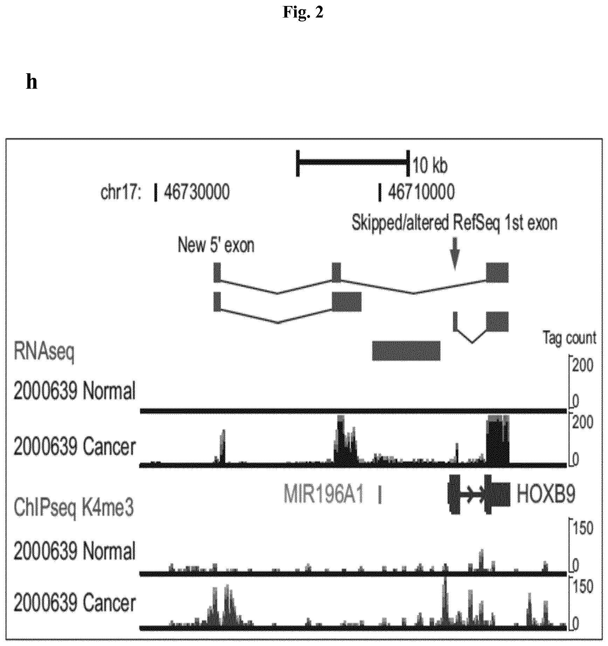

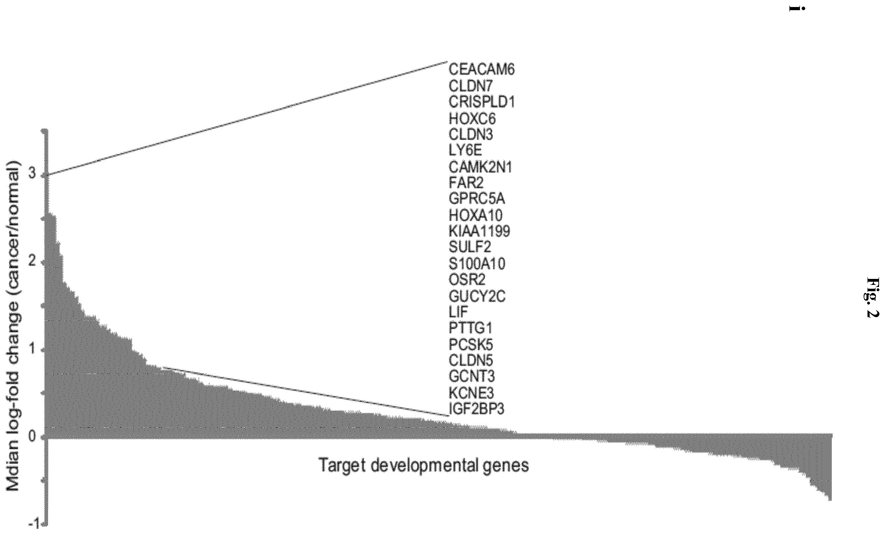

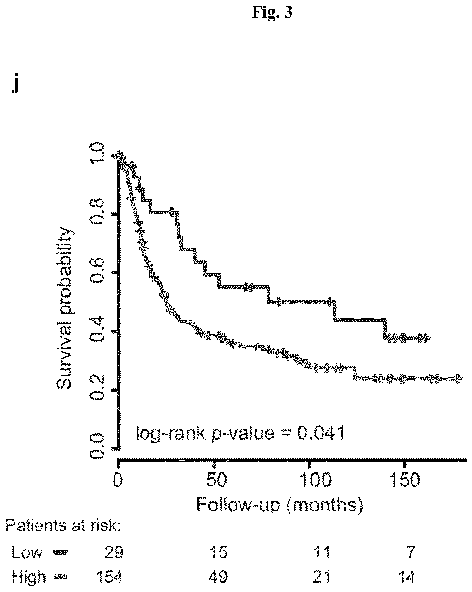

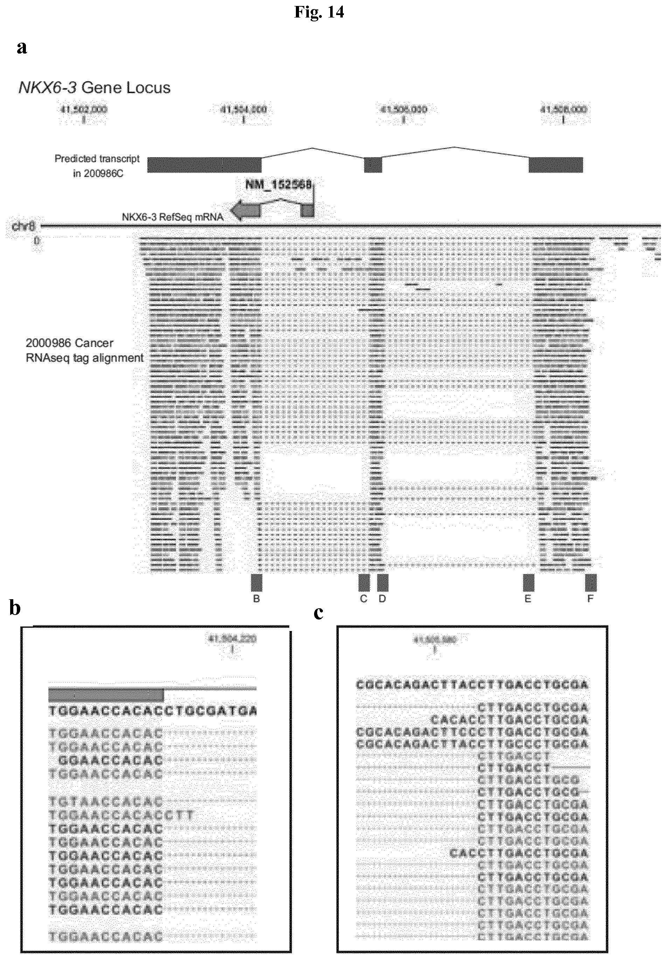

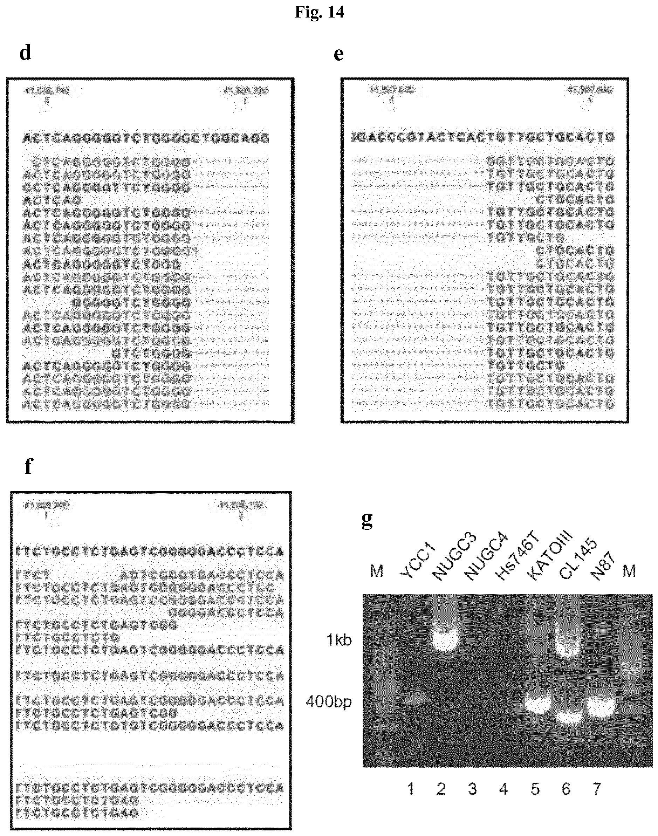

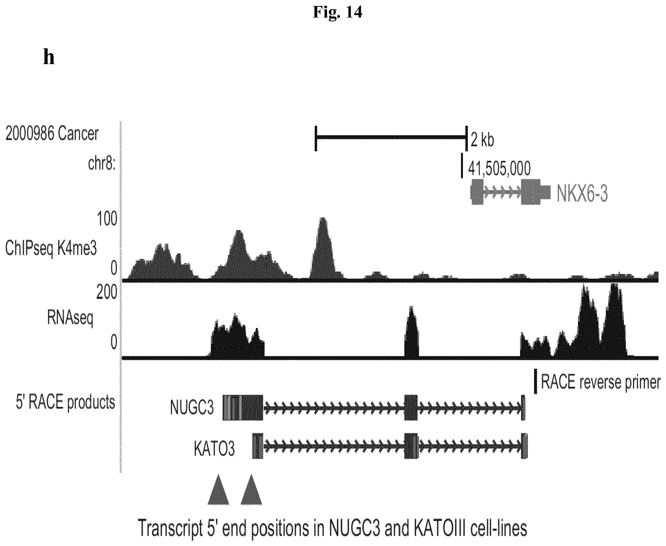

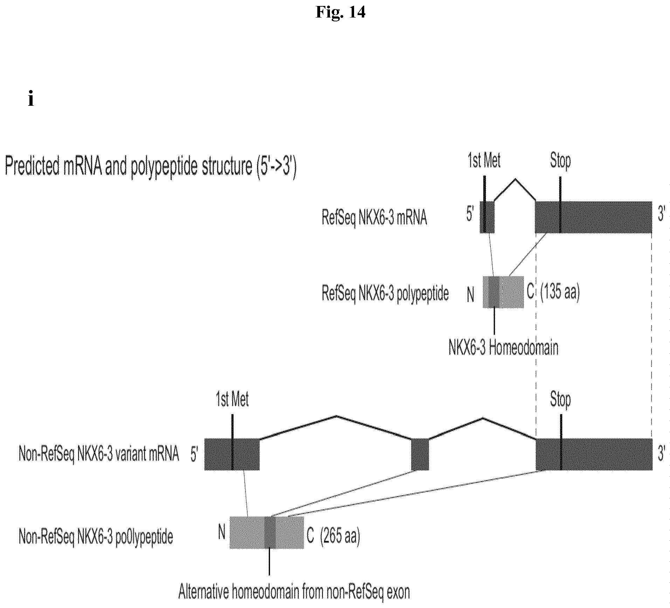

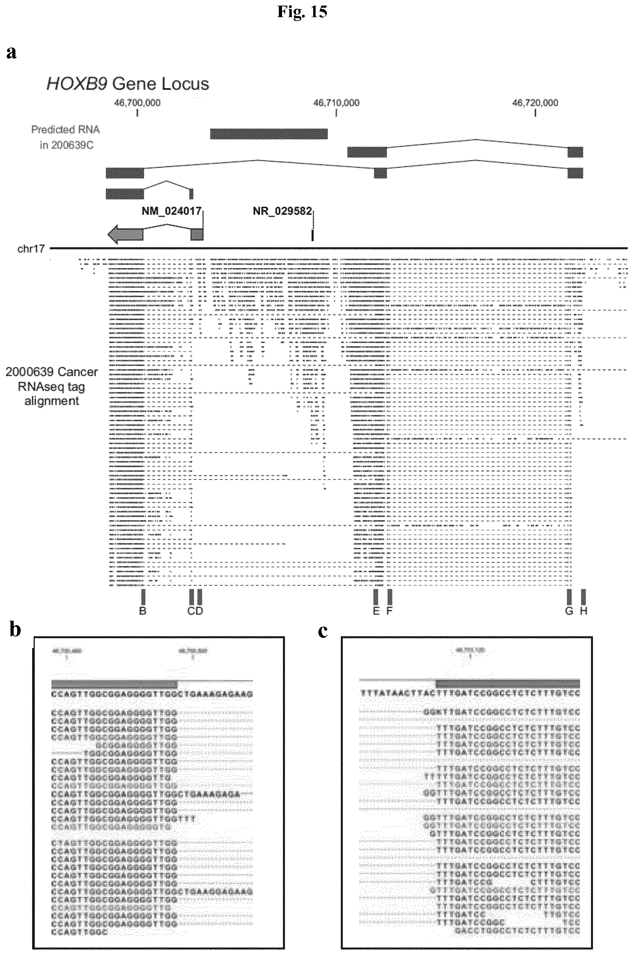

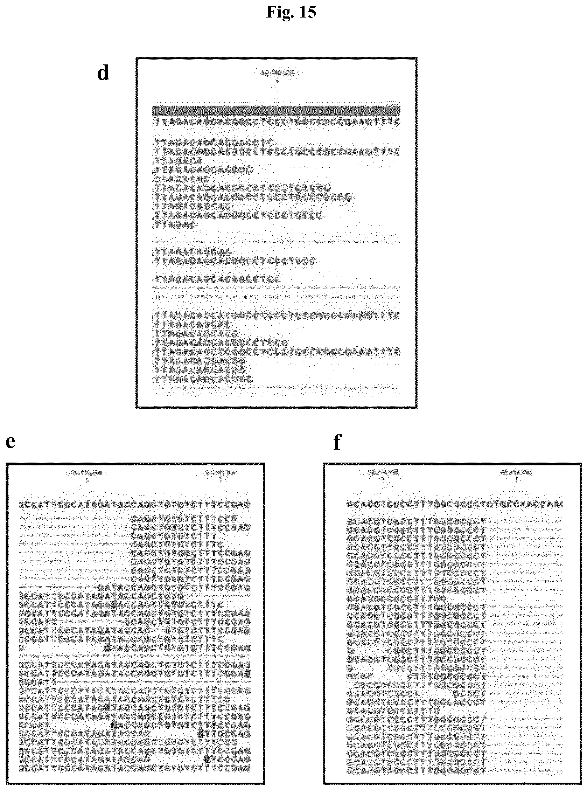

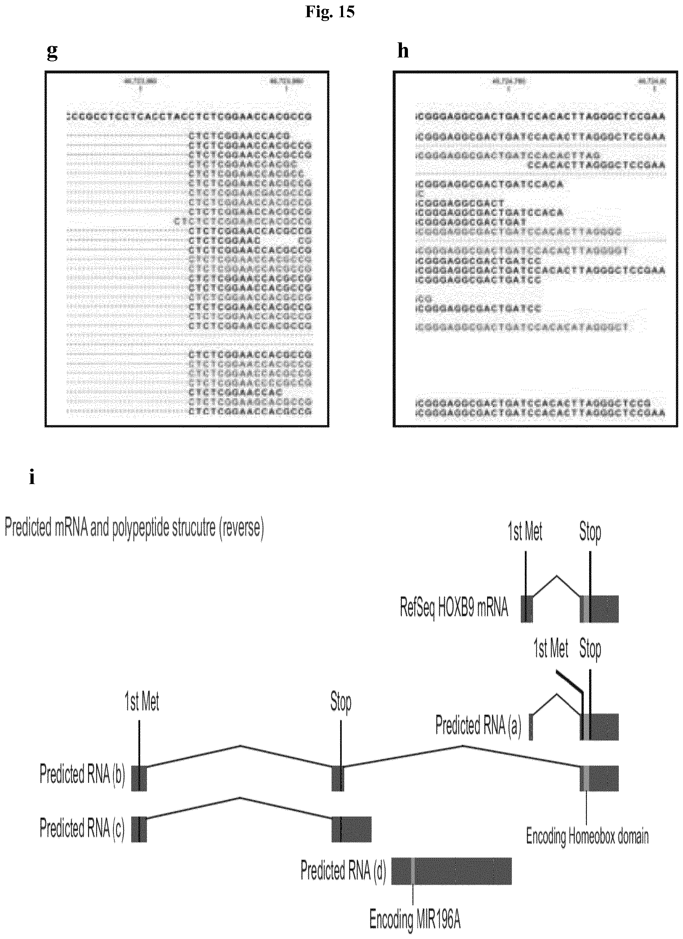

**Figure 2****. Cancer-associated Promoters in GC.** (a) Cancer-associated promoters are frequently associated with non-RefSeq TSSs ("cryptic promoters"). Cryptic promoter proportions associated with all promoters ("total") and promoters lost in cancer ("loss") are provided as references. Cancer-associated promoters are also associated with expressed non-RefSeq transcripts from RNAseq data (rightmost numbers). (b) Heatmap showing expression status of non-RefSeq transcripts exhibiting >4-fold expression changes between normal tissues and gastric tumors (FPKM; fragments per kilobase of transcript per million mapped reads). The transcripts are associated with 192 cancer-associated promoters. (c) GREAT analysis demonstrating enriched gene categories for cancer-associated promoters. All enriched terms with p<6×10-6from the original GREAT output are listed. (d, e) Cryptic promoter-driven *MET* expression. RNAseq and H3K4me3 tracks are shown. (e) Close-up view of the cryptic promoter region, showing representative "split" RNAseq tags confirming linkage of the promoter to downstream *MET* exons. (f) MET functional domains. The predicted cryptic-promoter driven transcript encodes an N-terminal truncated protein lacking the Sema domain. (g) Cryptic promoter-driven *NKX6-3* expression. RNAseq and H3K4me3 tracks are shown. RNAseq alignments are provided in Figure 14. (h) Cryptic promoter-driven *HOXB9* expression. RNAseq alignments are provided in Figure 15. (i) Expression levels of H3K4me3-marked genes between GCs (n=185) and matched normals (n=89). A significant proportion of genes are upregulated in GC (up-regulated genes = 143; Total target genes =218; p = 5.68x10-6). (j) Survival analysis comparing patient groups with GCs exhibiting high and low expression of genes driven by cancer-associated promoters. Kaplan-Meier survival analysis of clusters within the Singaporean cohort (total n = 183) with "high" (n = 154) and "low" (n = 29) enrichment of the target gene signature. The signature is prognostic in this cohort (log-rank p-value: 0.041), with worse prognosis observed for higher enrichment of the signature (H.R. (95% C.I.): 1.78 (1.02-3.13); p =0.044).

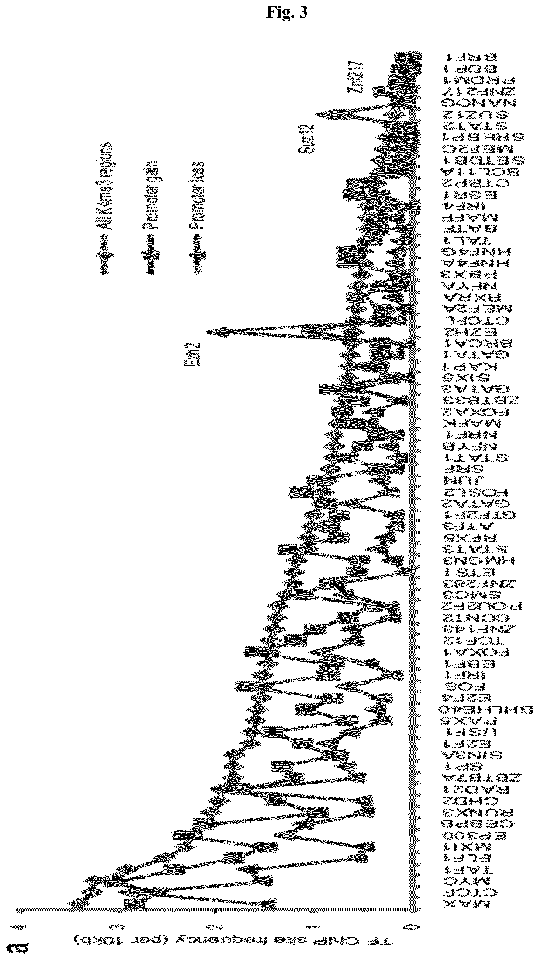

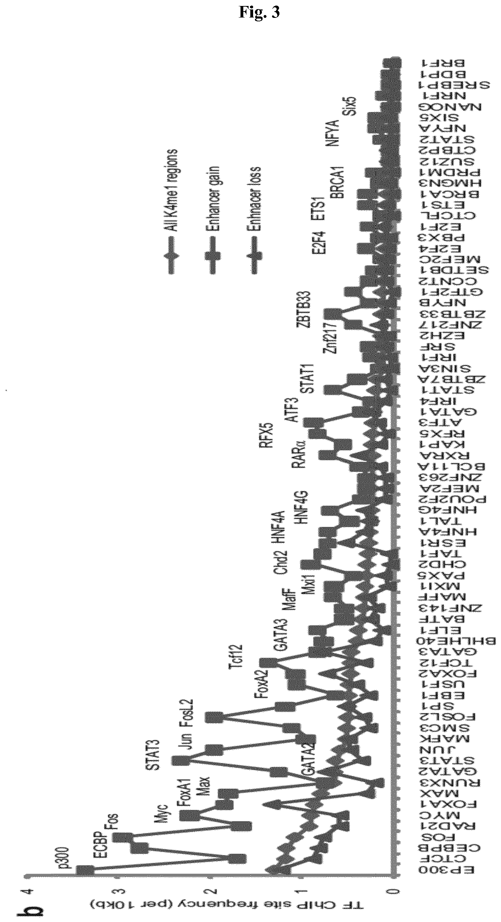

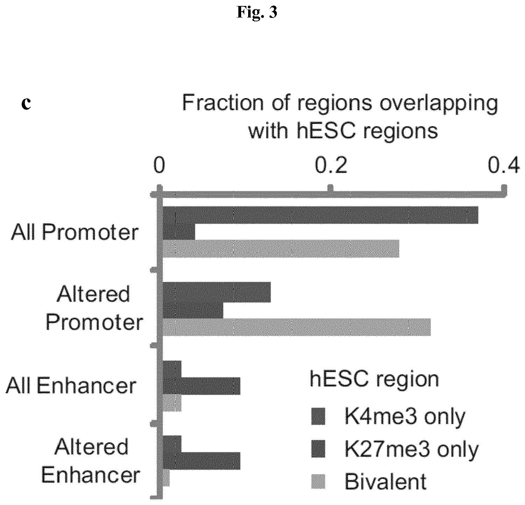

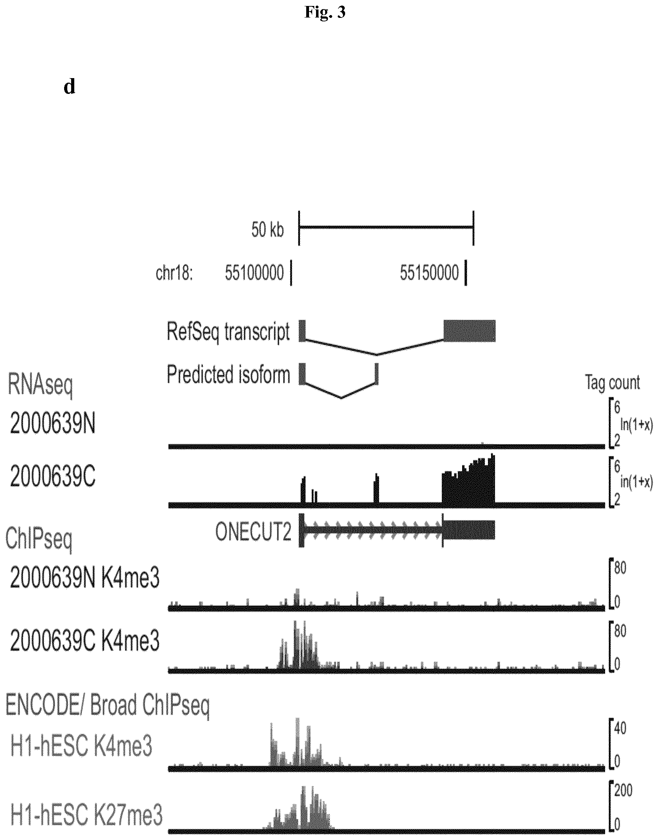

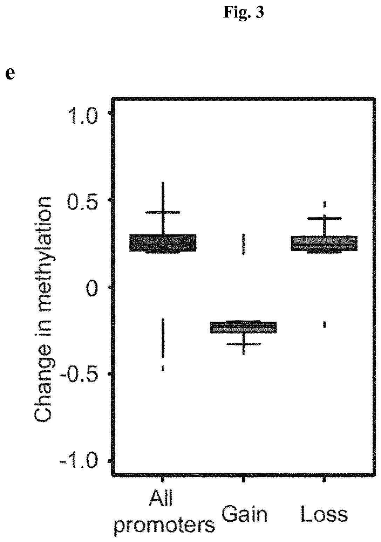

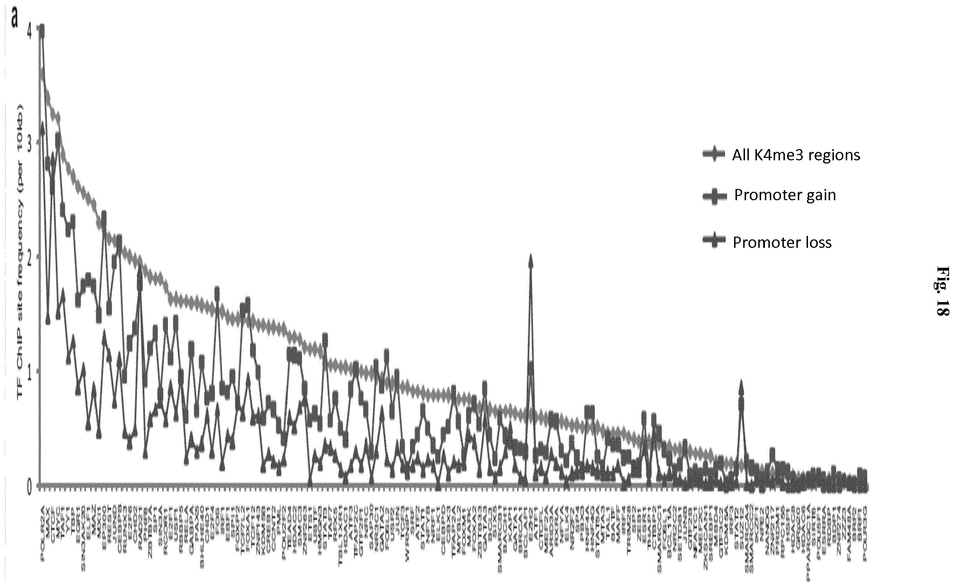

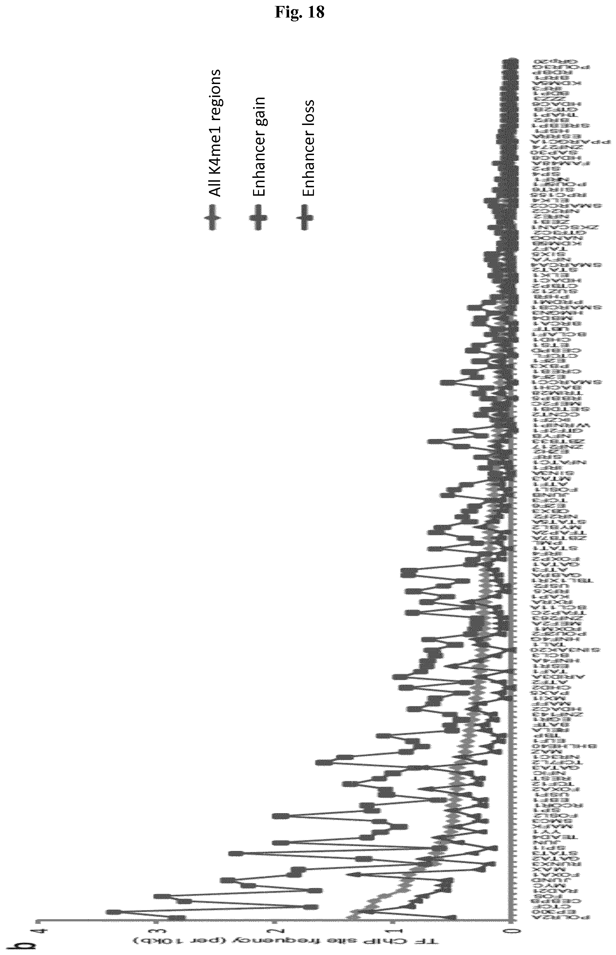

**Figure 3****. Binding Site Analysis of Cancer-Associated Regulatory Elements.** (a) Frequency of ENCODE-defined transcription factor binding sites (TFBSs) overlapping with cancer-associated promoters (gained and lost). Values are presented as the number of TFBS per 10 kb coverage. TFs were sorted according to their frequency in all H3K4me3-defined promoter sets. EZH2, SUZ12 and ZNF217 binding sites are enriched (p<0.05). The complete TF list is presented as Figure 18. (b) TFBS frequency in cancer-associated enhancer regions. (c) Overlap analysis between ESC-defined univalent (H3K4me3 only, H3K27me3 only) or bivalent (H3K4me3 and H3K27me3) regions and GC promoters (all and cancer-associated) and GC enhancers (all and cancer associated). Cancer associated GC promoters exhibit an elevated proportion of bivalent regions, exceeding univalent regions (p < 2.2x10-16). (d) Genome browser view of the *ONECUT2* locus as a representative cancer-associated promoter overlapping with an ESC-defined bivalent region. (e) Box plot depicting DNA methylation changes in all promoters and cancer-associated promoters (gained or lost). P-values (Wilcoxon test) are: p=7x10-48 (All promoters vs gained promoters); p=0.48 (All promoters vs lost promoters); p=5.37x10-41 (Gained promoters vs lost promoters).

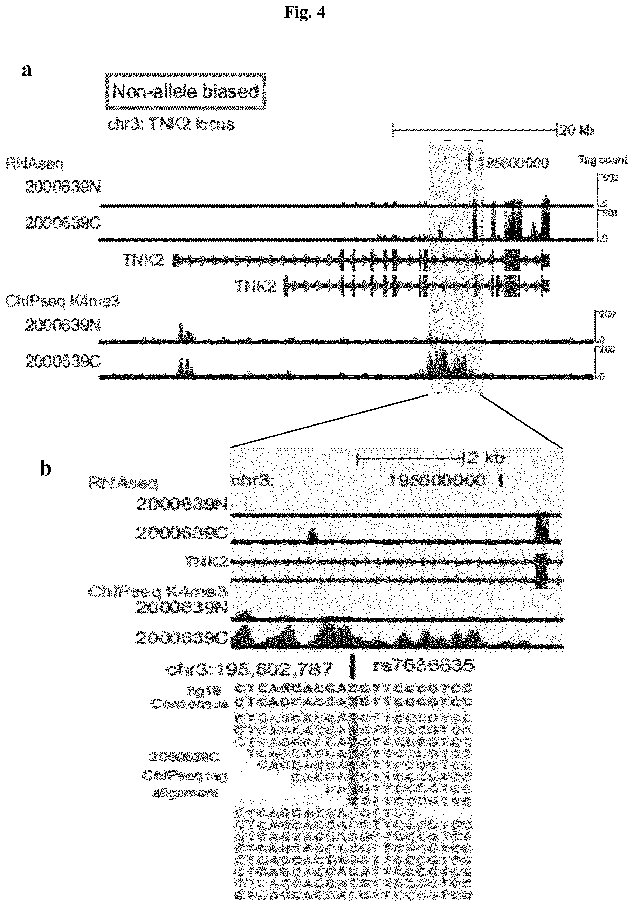

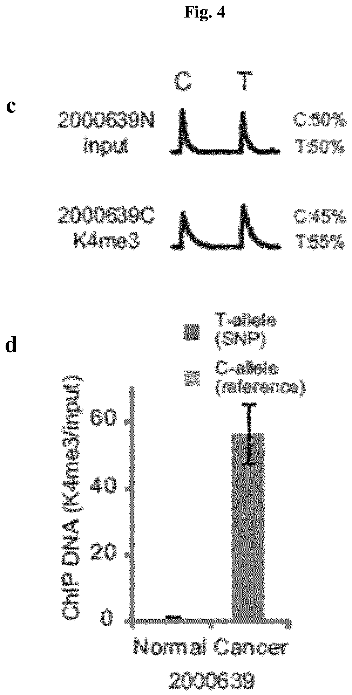

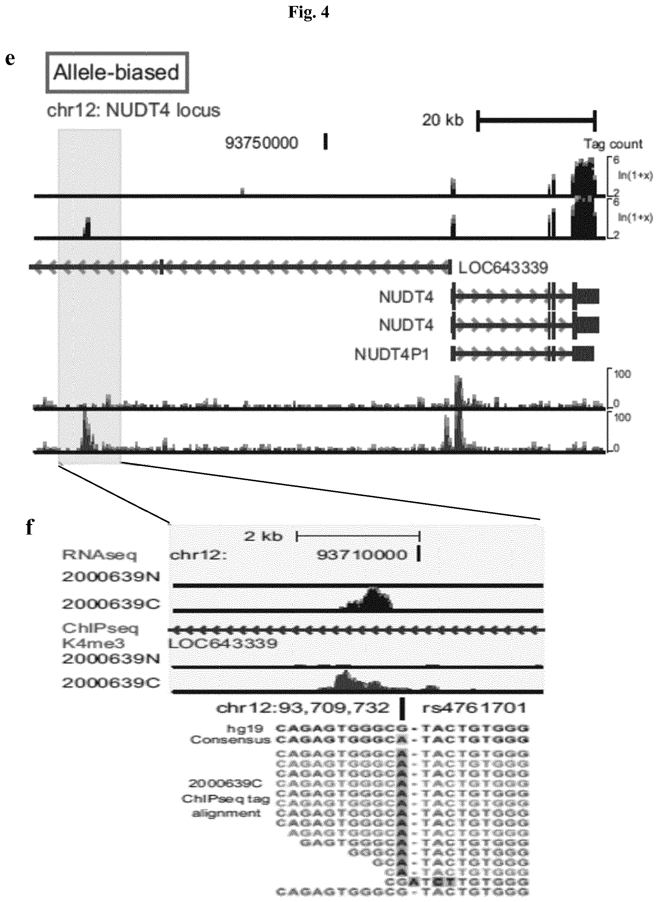

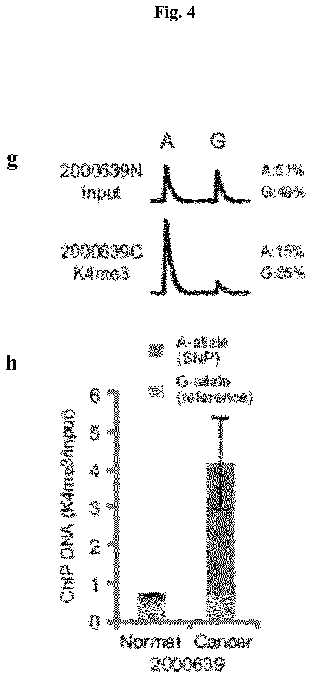

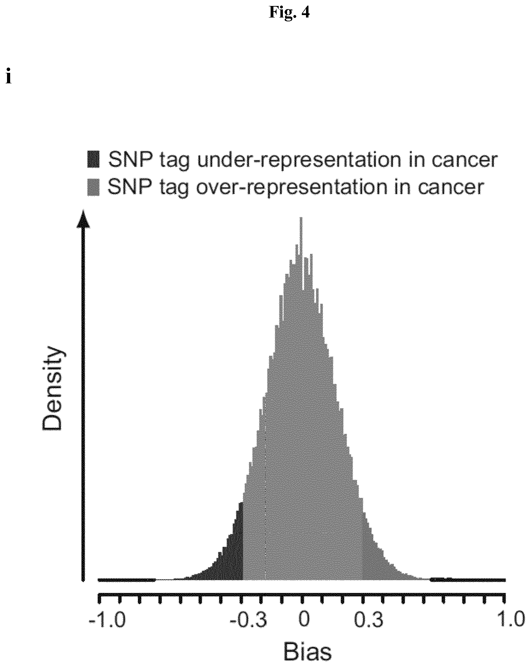

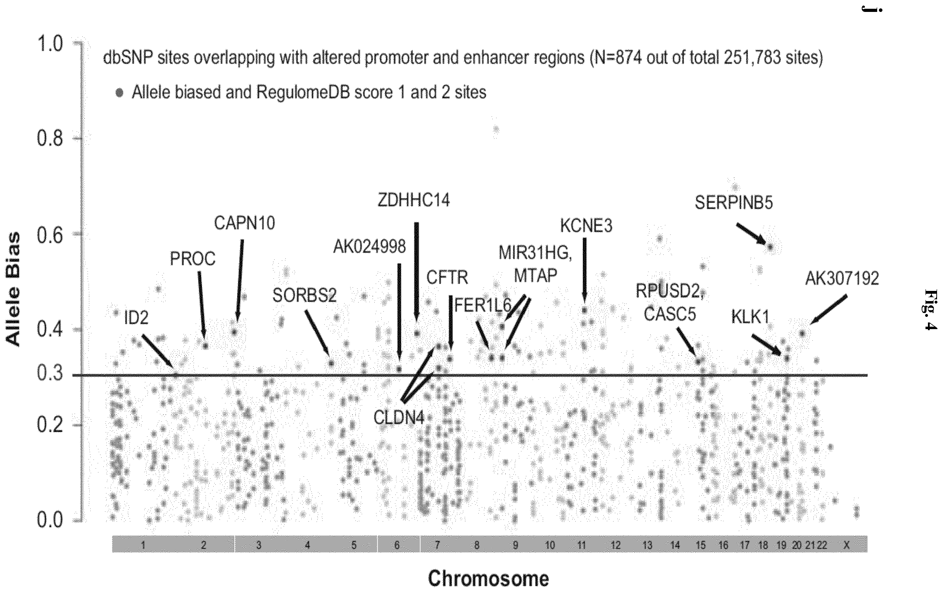

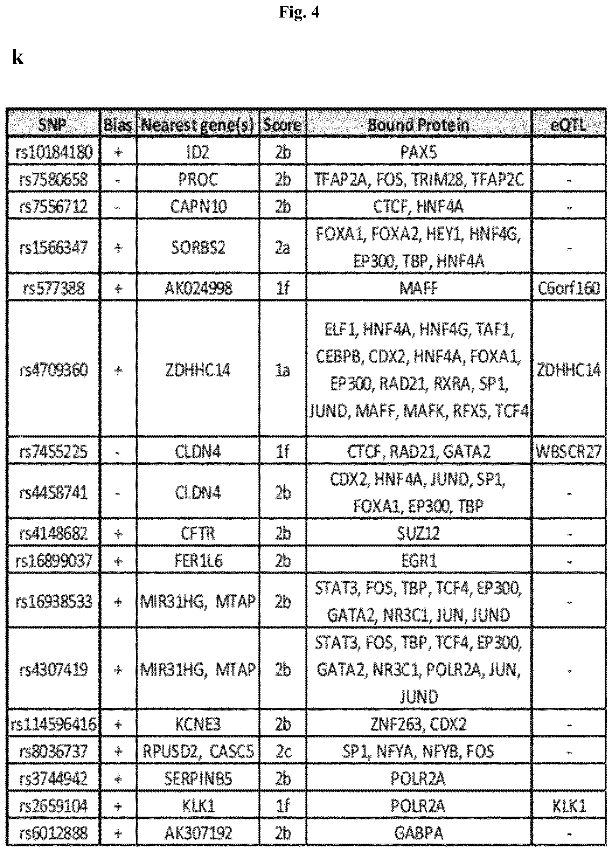

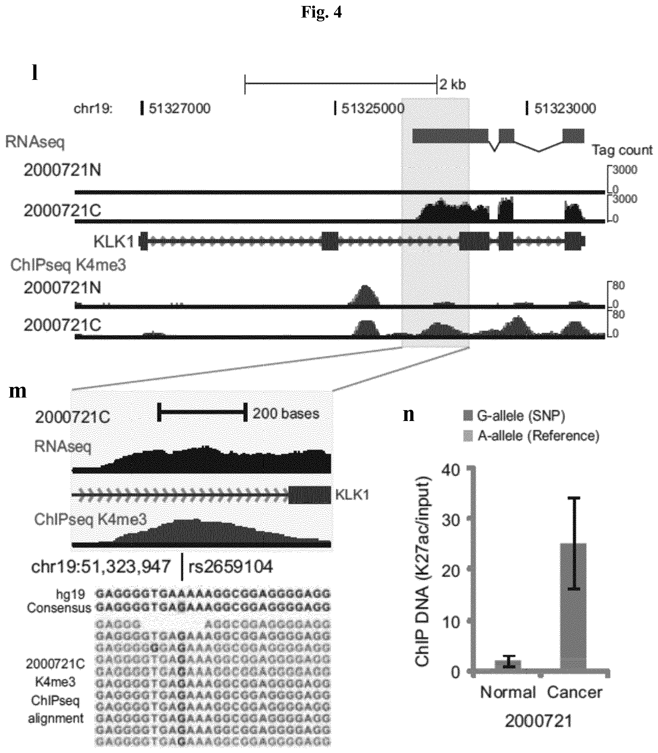

**Figure 4****. Allele-specific Regulatory Elements Associated with GC.** (a-d) Non-allele biased and (e-h) Allele-biased regulatory elements. (a-b) Genome browser view of the *TNK2* locus showing RNAseq and H3K4me3 tracks. (b) provides a close-up view and visualization of H3K4me3 sequence tags and SNPs. A comparable proportion of reference (C) and rs7636635 (T) SNPs are observed in the H3K4me3-enriched sequence reads. (c) Genotyping of normal tissue confirms equivalent allele heterozygosity in normal tissues and H3K4me3-enriched sequence tags from tumors. (d) Quantitative PCR-pyrosequencing confirms lack of H3K4me3 signal in normal tissue, and an equal proportion of reference (C) and rs7636635 (T) allele containing H3K4me3-enriched sequence reads from tumors. (e, f) Genome browser view of the *NUDT4* locus showing RNAseq and H3K4me3 tracks. (f) provides a close-up view and visualization of H3K4me3 sequence tags and SNPs. A bias favoring a higher proportion of rs4761701 SNPs (A) over the reference allele (G) is observed. (g) Genotyping of normal tissue confirms equivalent allele heterozygozity in normal tissues but a bias towards the rs4761701 SNP (A) in H3K4me3 sequence tags from tumors. (h) Quantitative PCR-pyrosequencing confirms minimal H3K4me3 signal in normal tissue, and an rs4761701 SNP-biased proportion of sequence tags over the reference allele in H3K4me3 signals from tumors. (i) Allele bias distribution across samples. Over- and under-representation of SNP tags in tumor tissues are marked in green and blue, respectively. (j) dbSNP sites mapping to altered promoter and enhancer regions. SNP sites are positioned according to their chromosomal position on the x-axis, and their corresponding allele bias levels along the y-axis. SNPs exhibiting allele bias (above blue horizontal line) and which are also predicted to affect protein binding based on RegulomeDB are marked in red. (k) Regulome DB predictions for allele-biased sites mapping to somatically altered regulatory elements. (l, m) Genome browser view of the *KLK1* locus showing RNAseq and H3K4me3 tracks. (m) provides a close-up view of H3K4me3 sequence tags and SNPs. A bias favoring a higher proportion of the known eQTL SNP rs2659104 (G) over the reference allele (A) is observed. (n) Quantitative PCR-pyrosequencing confirms minimal H3K4me3 signal in normal tissue, and rs2659104 SNP-biased proportions of sequence tags over the parental allele in H3K4me3 signals from tumors.

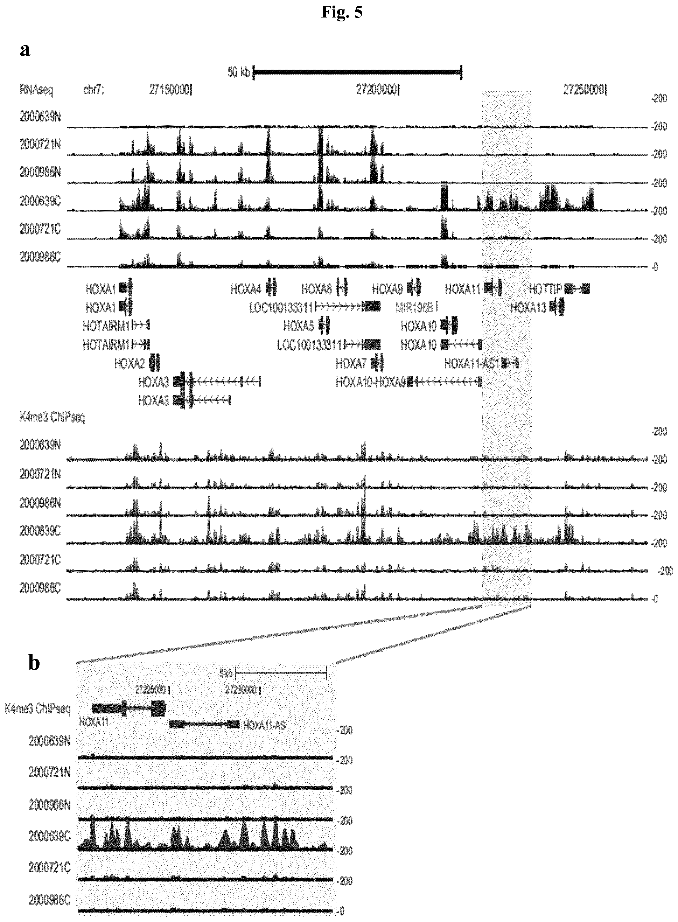

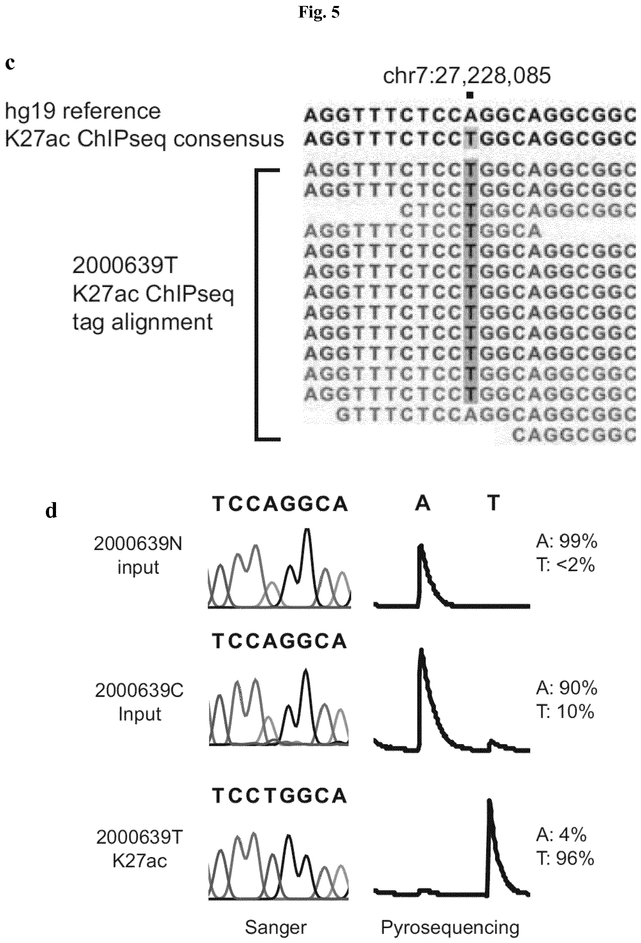

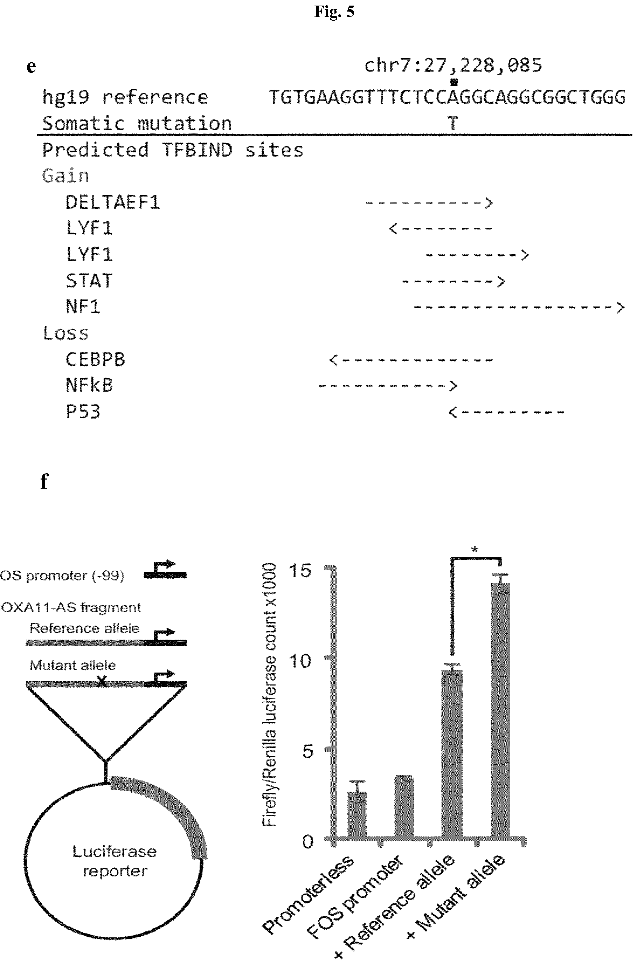

**Figure 5****. Somatic Regulatory Mutations in GC**. (a) Genome browser view of the *HOXA* locus. (top) RNAseq data. *HOXA* genes distal to (and including) *HOXA11* are expressed in GC 2000639 in a cancer-specific fashion. (bottom) H3K4me3 signals confirm an altered chromatin domain in this region. (b) Close-up view of the *HOXA11* region. Cancer-specific acquisition of H3K4me3 in GC 2000639 is observed. (c) H3K27ac sequence tag alignments predict the presence of a T allele at position chr7:27,228,085 while the reference (hg19) exhibits an A at this site. This site does not correspond to a known dbSNP. (d) Sanger sequencing and pyrosequencing validation of genotypes in input normal DNA, input tumor DNA, and H3K27ac-enriched ChIPseq DNA. The normal sample is homozygous for the A allele, while the cancer has a small T signal at this position (estimated allele frequency 10%). In contrast, H3K27acenriched DNA exhibits a high proportion of T alleles (96%). (e) TF site predictions by TFBIND for reference and somatically mutated alleles. LYF1, STAT and NF1 sites are predicted to be gained, while CEBP, NFKB and p53 sites are predicted to be lost. (f) Luciferase reporter assays measuring regulatory activity of wild-type and mutant alleles. DNA containing the mutant allele provides increased transcriptional activity (* p=1.1x10-4). Experiments were performed in KATO-III GC cells.

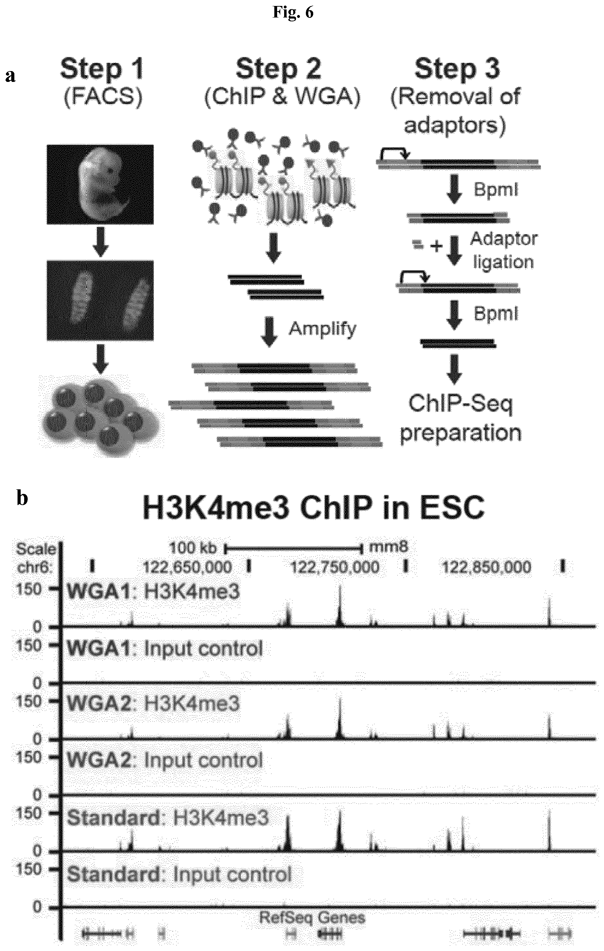

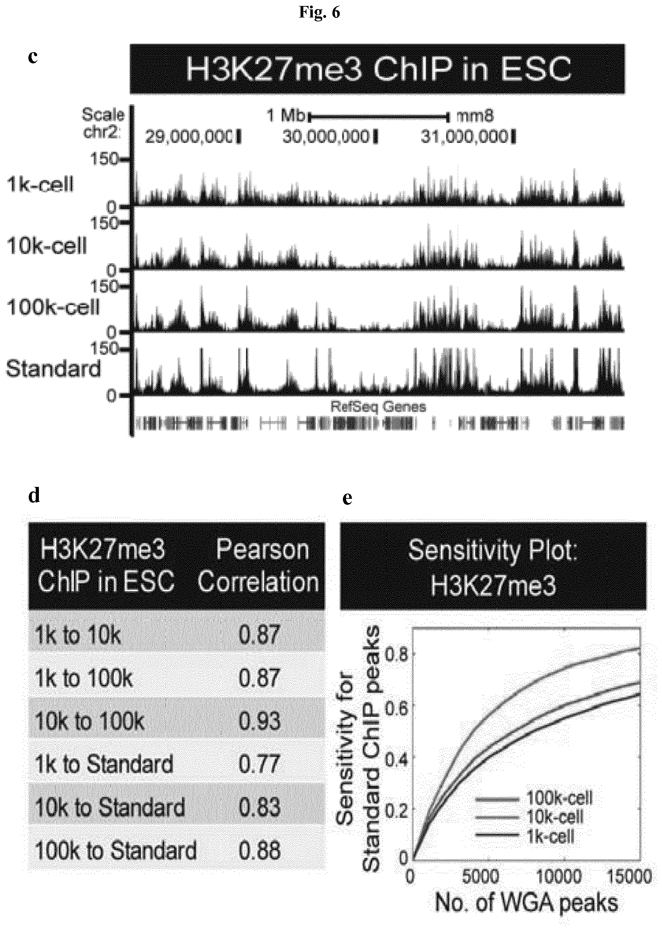

**Figure 6****. 1,000-cell Scale Nano-ChIPseq Validation from** Ng et al. (2013) Developmental Cell. Comparison between small-scale ChIPseq (ie Nano-ChIPseq) and Standard ChIPseq.

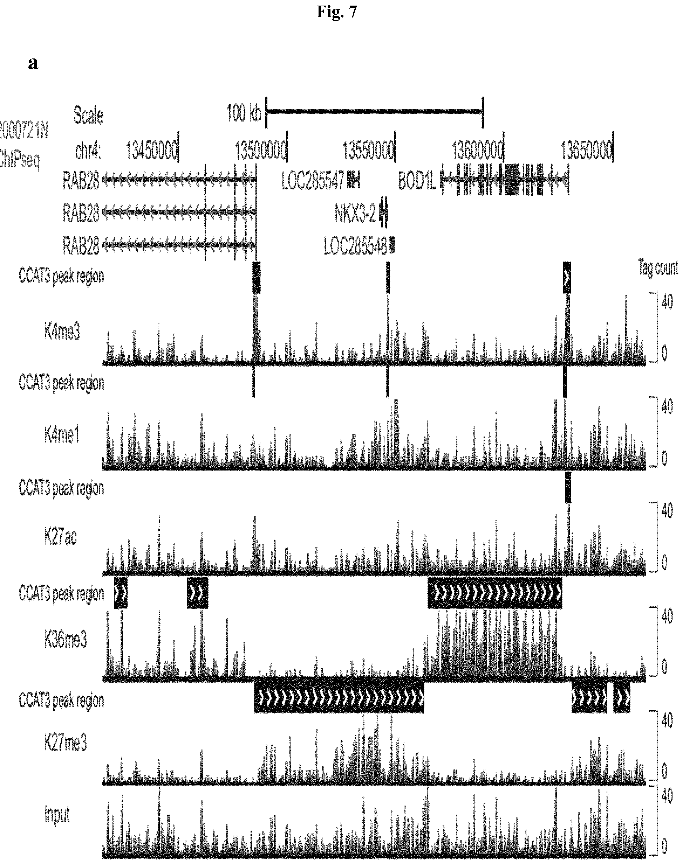

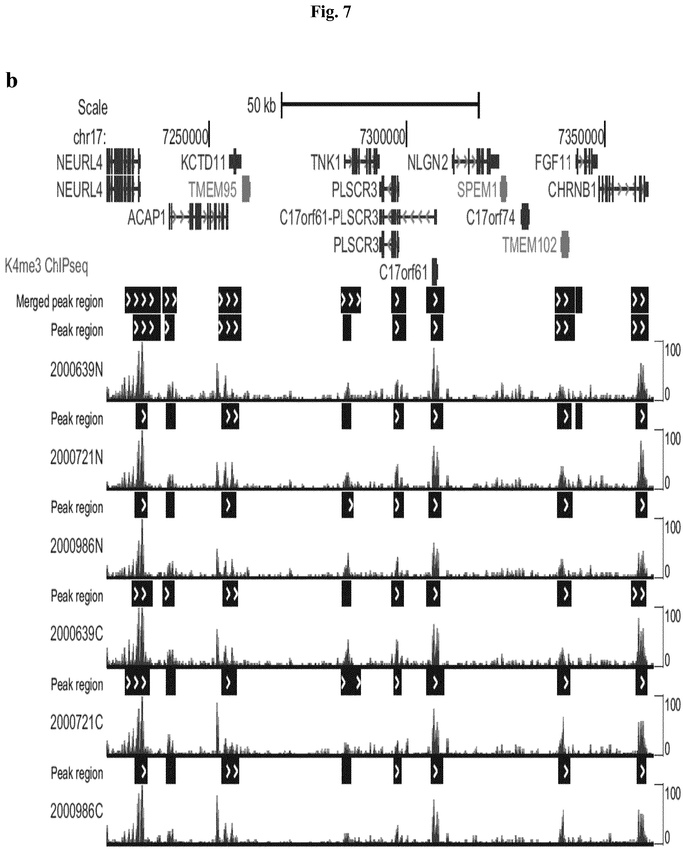

**Figure 7****. Nano-ChIPseq Peak Calling and Peak Merging.** (a) Representative UCSC browser view of Nano-ChIPseq tag density for 5 histone modifications in patient 2000721 normal (2000721N). Peak regions are indicated above each ChIPseq track. The gene track shows RefSeq transcripts. (b) H3K4me3 peak profile of 6 tissue samples. Merged peak regions are indicated below the RefSeq transcript track.

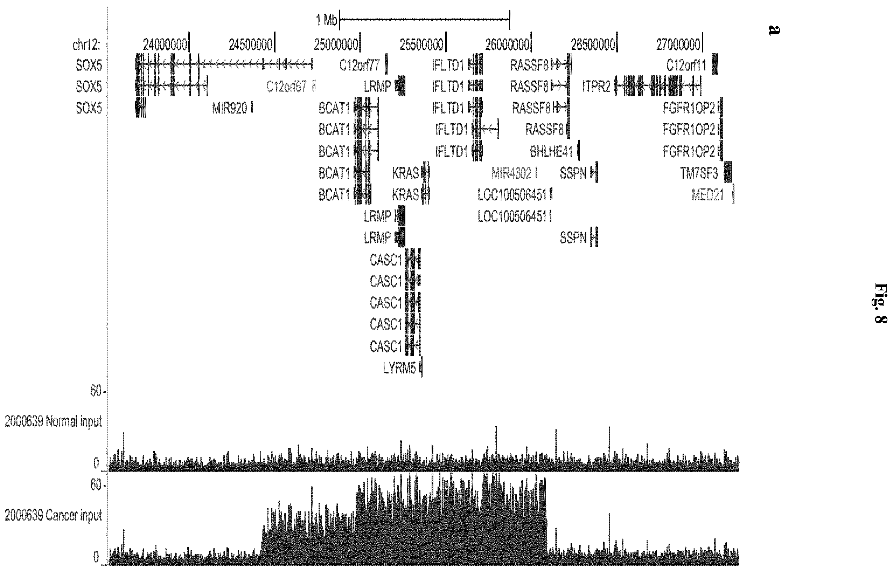

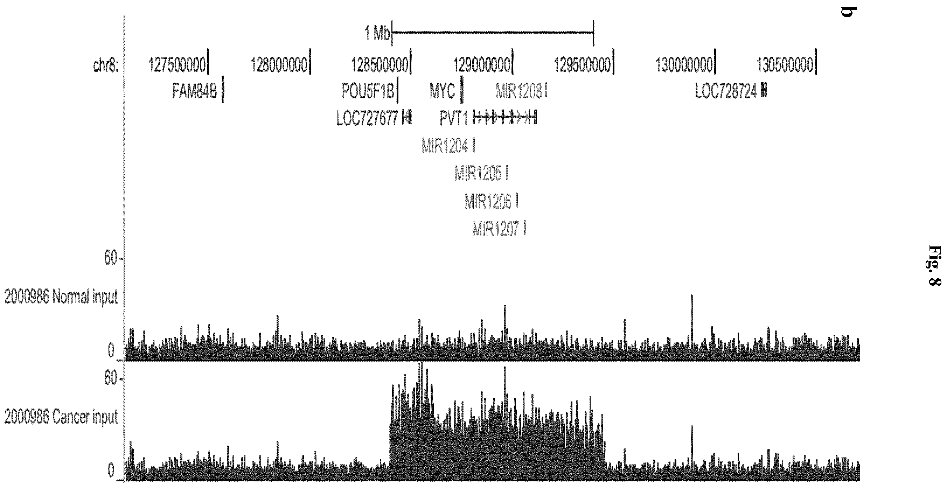

**Figure 8****. Identification and Removal of Amplified Regions.** Representative views of amplified loci around the *KRAS* locus in tissue 2000639C (a) and *MYC* in tissue 2000986C (b). Regions exhibiting an abundance of sequence tags in the cancer input DNAs were removed from subsequent analysis.

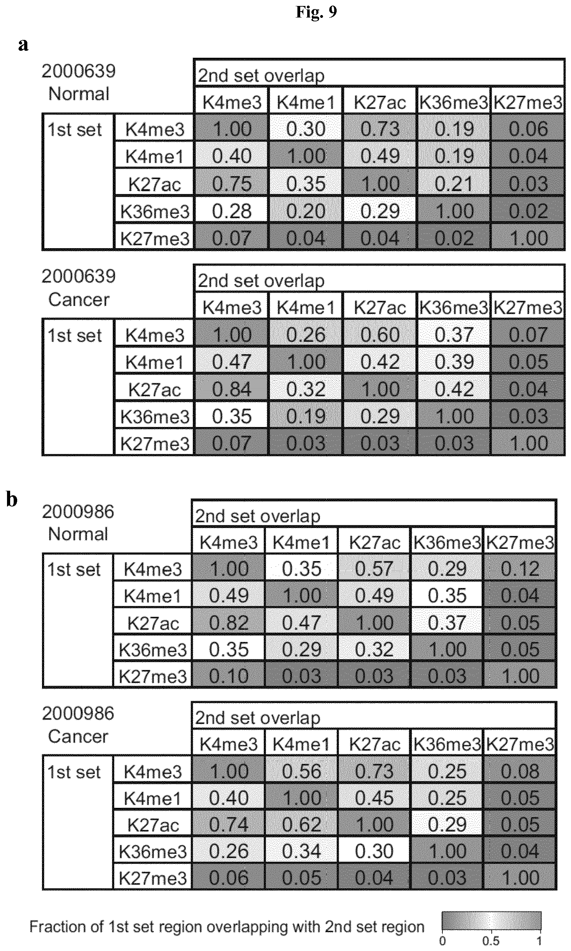

**Figure 9****. Overlap of Chromatin Marks in GC/Normal Pairs.** Overlap of chromatin mark regions in normal (top) and cancer tissue (bottom) for sample pairs 2000639 (a) and 2000986 (b). Numbers represent the fraction of 1st set regions (vertical) overlapping with the 2^{nd} set regions (horizontal). White = exclusive, dark gray = overlapping.

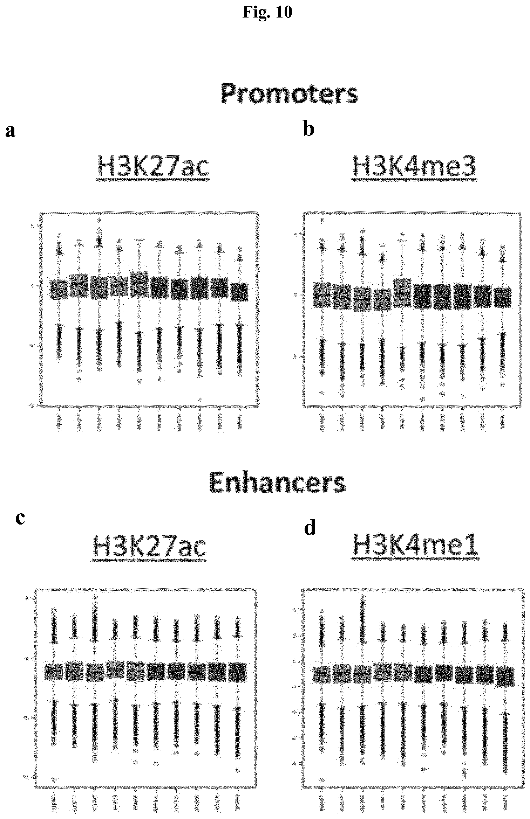

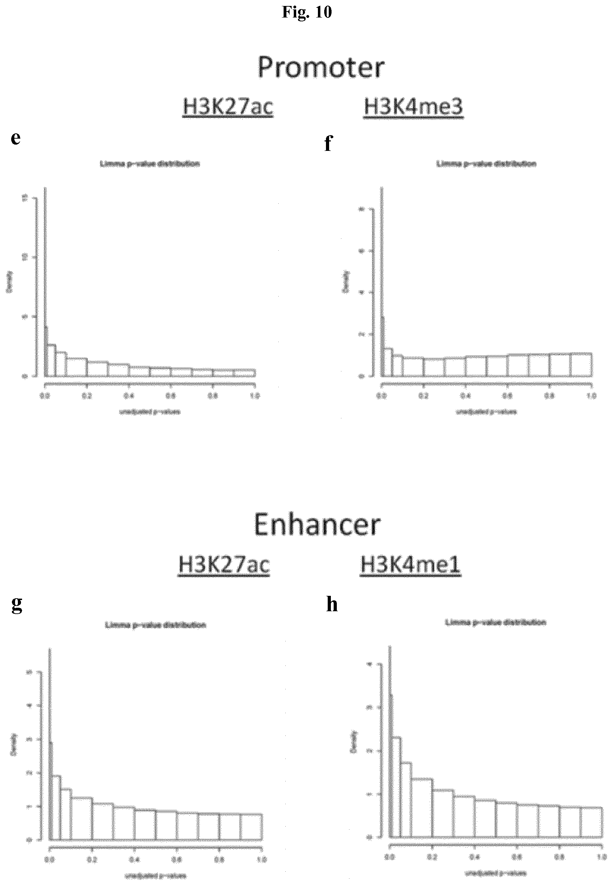

**Figure 10****. Nano-ChIPseq RPKM Pre-processing,** (a-d) Boxplots of log transformed ChIPseq FPKM values (after ComBat normalization) for promoter and enhancer regions. (e-h) Raw p-value distributions from linear model fitting.

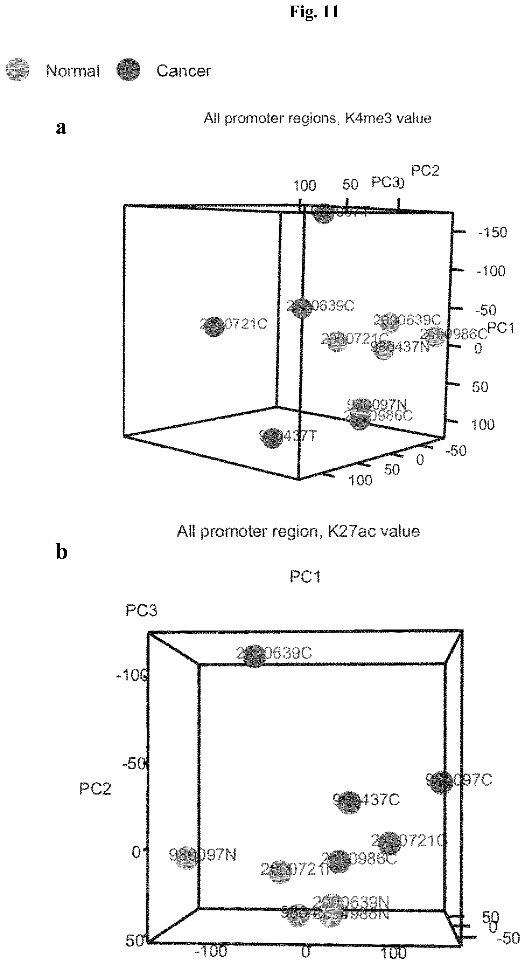

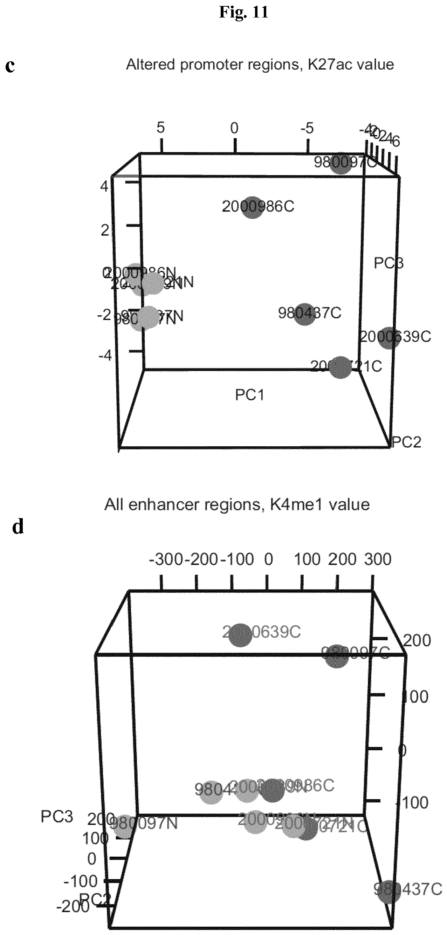

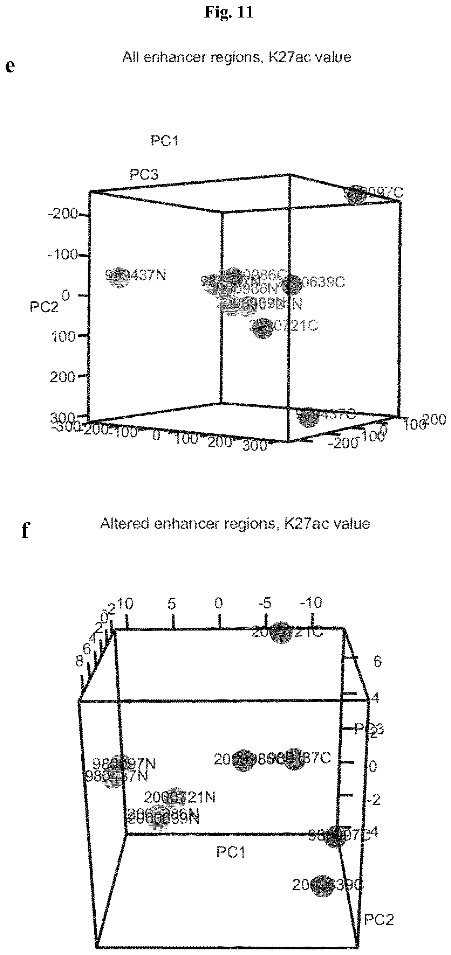

**Figure 11****. PCA plots of GCs and Normal Tissues.** (a,b) and (d,e) PCA plot of GCs (purple) and normal (blue) tissues using (a) all identified H3K4me3 (promoter) and (b) associated H3K27ac (activity); or (d) all identified H3K4me1 (enhancer) and (e) associated H3K27ac regions. (c and f) PCA plot using H3K27ac regions exhibiting somatic alterations.

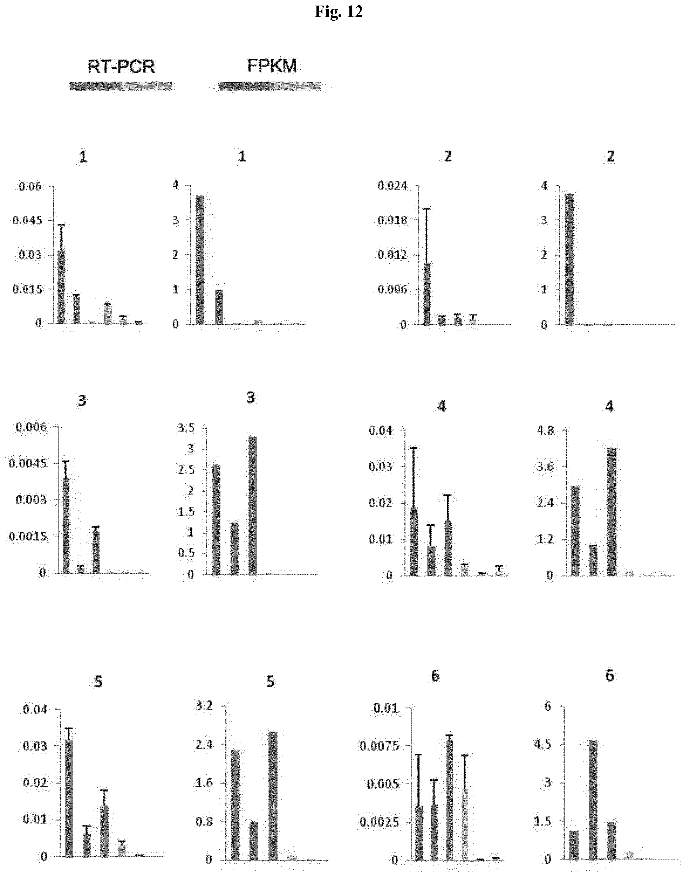

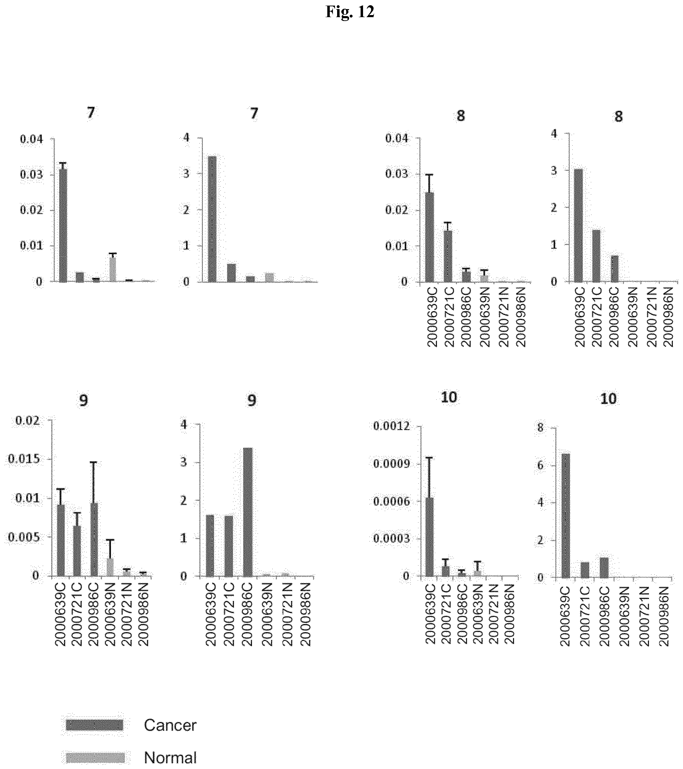

**Figure 12****. RT-qPCR Validation of Non-RefSeq Transcripts.** RT-qPCR validation results for 10 non-RefSeq transcripts in GCs and normal tissues, compared to FPKM values derived from RNAseq analysis.

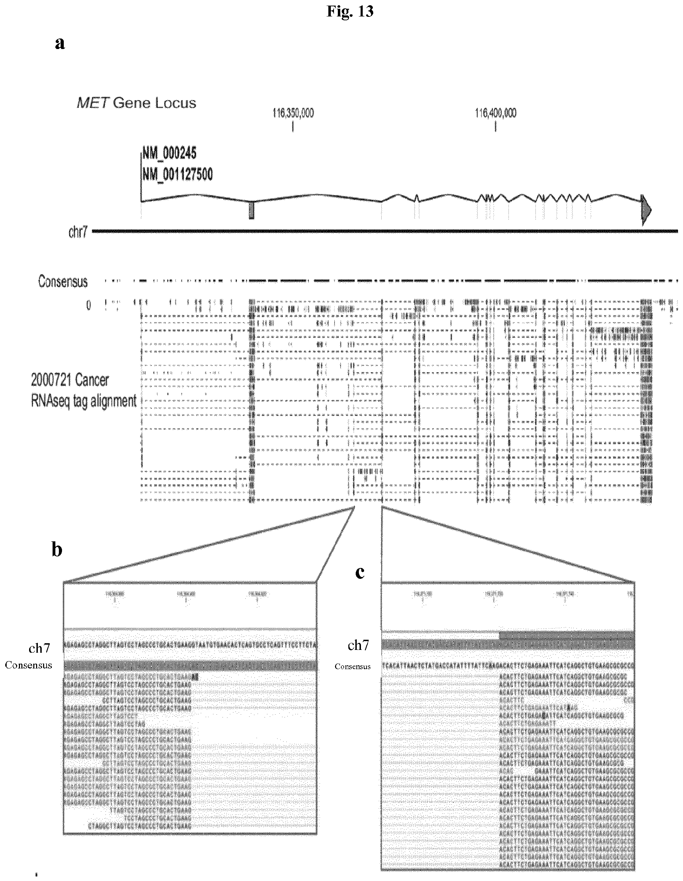

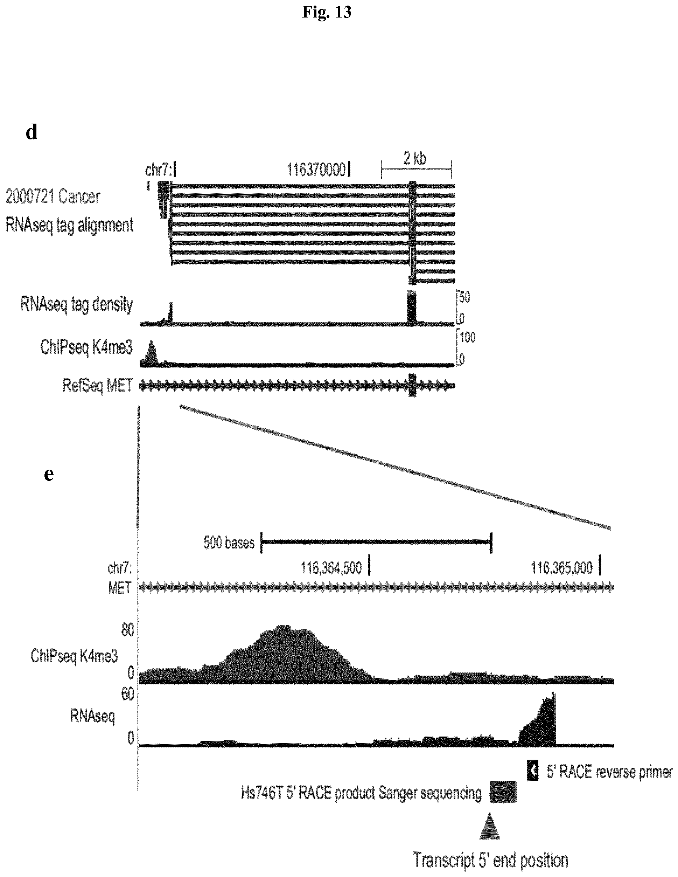

**Figure 13****. *MET* Gene RNAseq Alignment.** (a) RNAseq tag alignments supporting exon-intron structures of expressed RNAs at the *MET* gene locus. *MET* RefSeq transcripts are indicated in the top annotation. (b,c) Close-up view of RNAseq alignments. Exon-intron boundaries for cryptic promoter-driven exons were confirmed by manual inspection of sequence tag alignments. (d) Visualization of *MET* cryptic promoter showing location of RNAseq tags and H3K4me3 enrichment. The downstream *MET* Refseq exon is shown. (e) 5' RACE analysis. Close-up cryptic promoter view with RNAseq and H3K4me3-enriched reads. The location of the 5' RACE primer is indicated, and the product of the 5' RACE product from the MET-expressing line Hs746T is shown.
**Figure 14****. *NKX6-3* RNAseq Alignment.** (a) RNAseq tag alignments supporting exon-intron structures of expressed RNAs. *NKX6-3* RefSeq transcript (NM152568) is shown in green, and predicted cryptic-promoter expressed mRNAs are indicated at the top. (b-f) Close-up view of RNAseq alignments. Exon-intron boundaries were confirmed by manual inspection of sequence tag alignments. (g) Gel photo of two distinct 5' RACE products from 7 GC cell-lines. (h) Genome browser view showing positions of 5' RACE fragments from NUGC3 (larger RACE product) and KATOIII (smaller RACE product) cell-lines. The location of the 5' RACE primer is indicated. Both products validate the expression of a 5' non-Refseq exon, where NUGC3 has a larger product. Transcript 5' ends are shown by the red arrows (i) Predicted mRNA and polypeptide structures. Location of the *NKX6-3* homeodomain is indicated based on the RefSeq database.
**Figure 15****. *HOXB9* LocusRNAseq Alignment.** (a) RNAseq alignments at the *HOXB9* locus. Multiple spliced RNA isoforms were predicted based on RNAseq alignments. (b-h) Exon-intron boundaries were confirmed by RNAseq tag alignments. (i) Coding potential of predicted mRNAs and microRNA precursors are indicated. The oncogenic miRNA *MIR196A* also falls in this region.

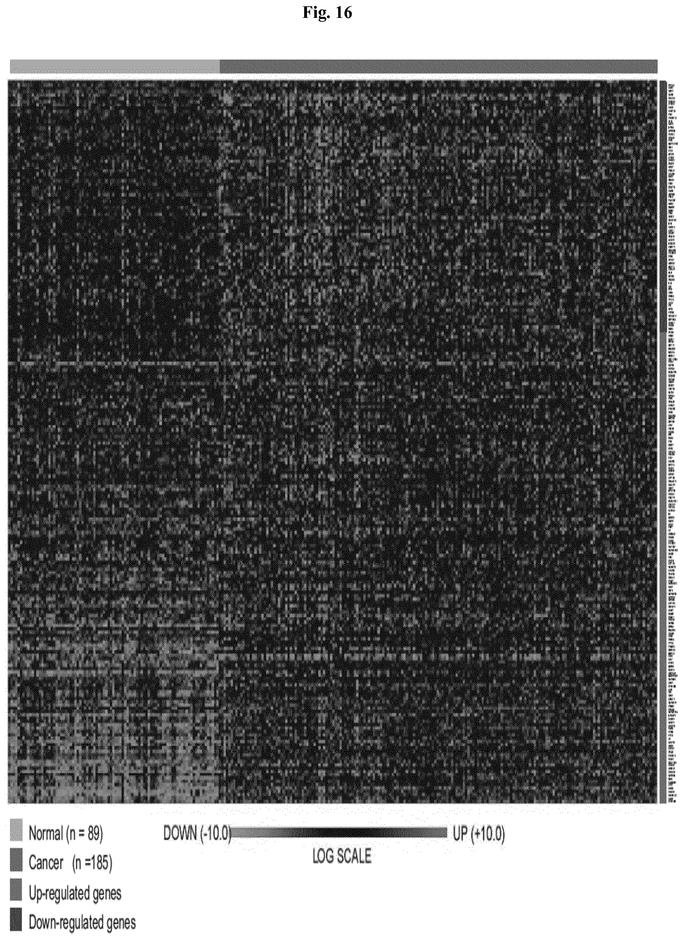

**Figure 16****. Microarray Validation Cohort.** Heatmap of log expression of upregulated H3K4me3-marked cancer-associated genes (n =218) in independent microarray data. "Cancer" (n = 185) and "Normal" (n = 89) samples are from Singapore. "Cancer" and "Normal" samples show distinct patterns of expression where the majority of H3K4me3-marked cancer-associated genes are upregulated in tumors.

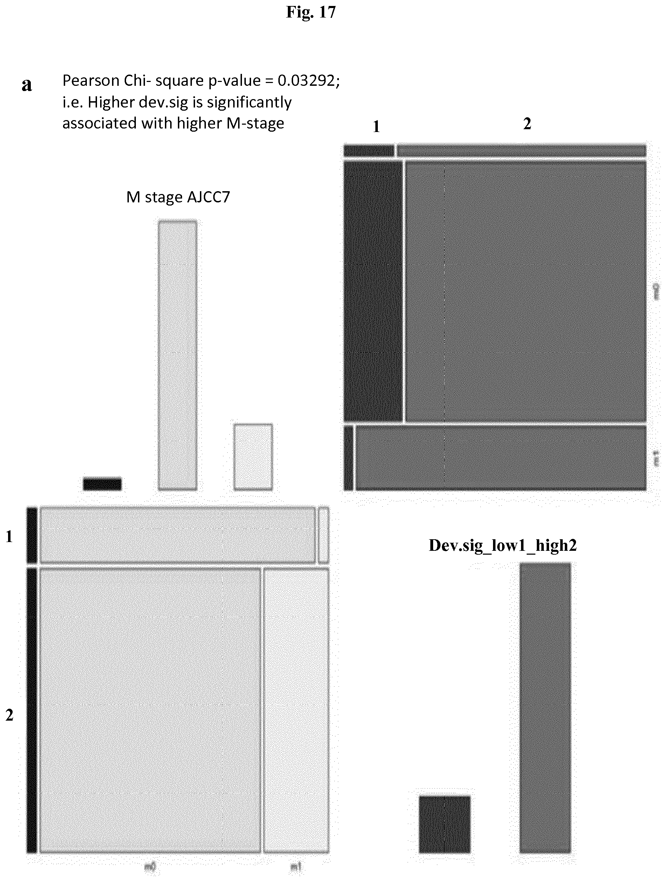

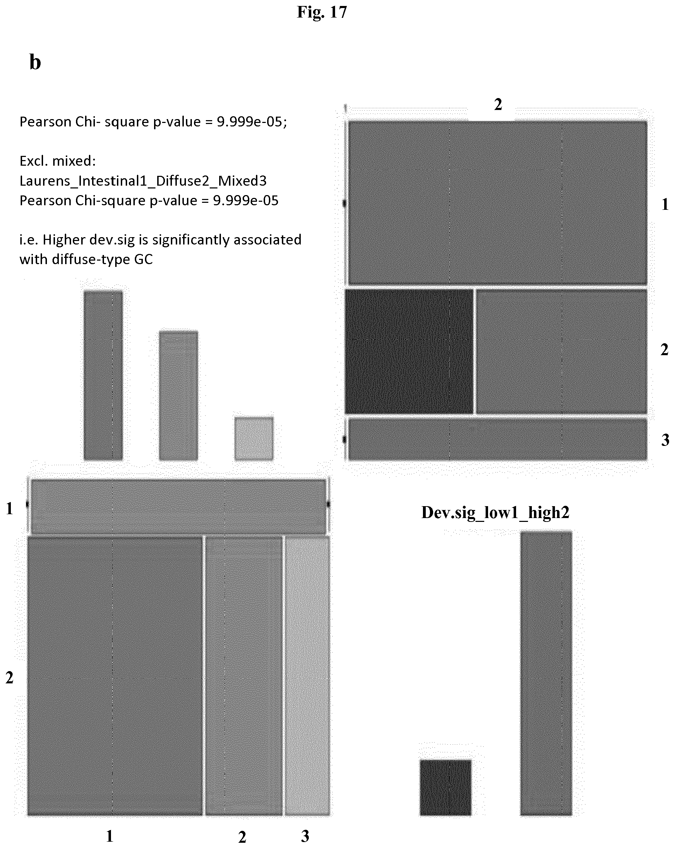

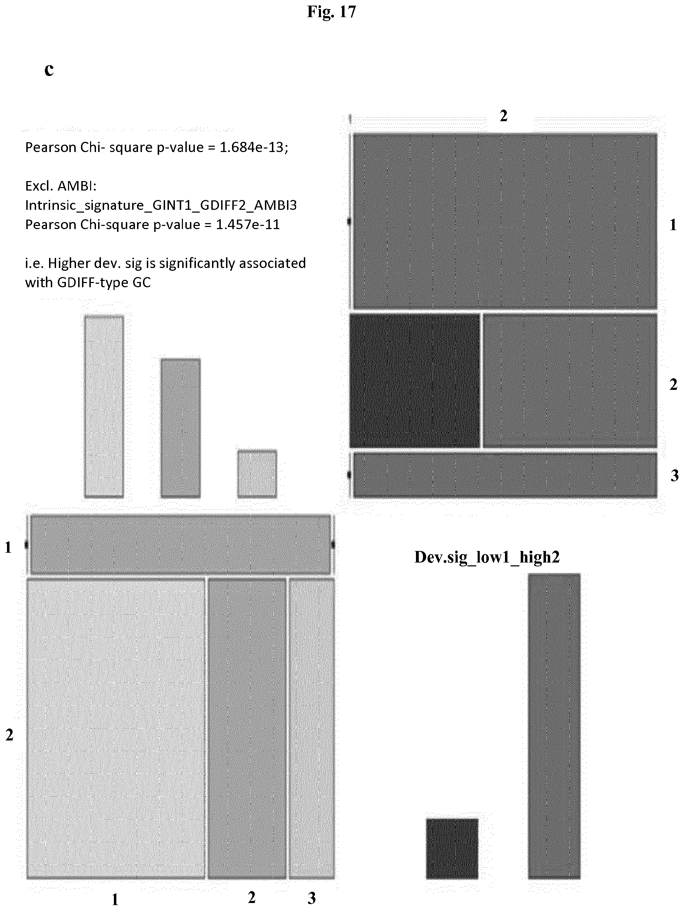

**Figure 17****. Association of Cancer-associated Genes with Clinicopathologic Features.** Mosaic plots of factors with significant association with H3K4me3-marked cancer-associated genes: (a) Mstage, (b) Lauren's histopathology, and (c) Intrinsic signature from Tan *et al.*14*.* Higher M-stage (p =0.033), diffuse Lauren's histopathology (p = 9.99x10-5), and GDIFF intrinsic signature classification (p=1.46x10-11) are significantly associated with high-expression of the H3K4me3-marked cancer-associated genes. Red: high-enrichment of signature; blue: low-enrichment of signature.
**Figure 18****. Overlap of GC Promoters and Enhancers with ENCODE Data.** (a) Frequency of TFBS in promoter regions. (b) Frequency of TFBS in enhancer regions.

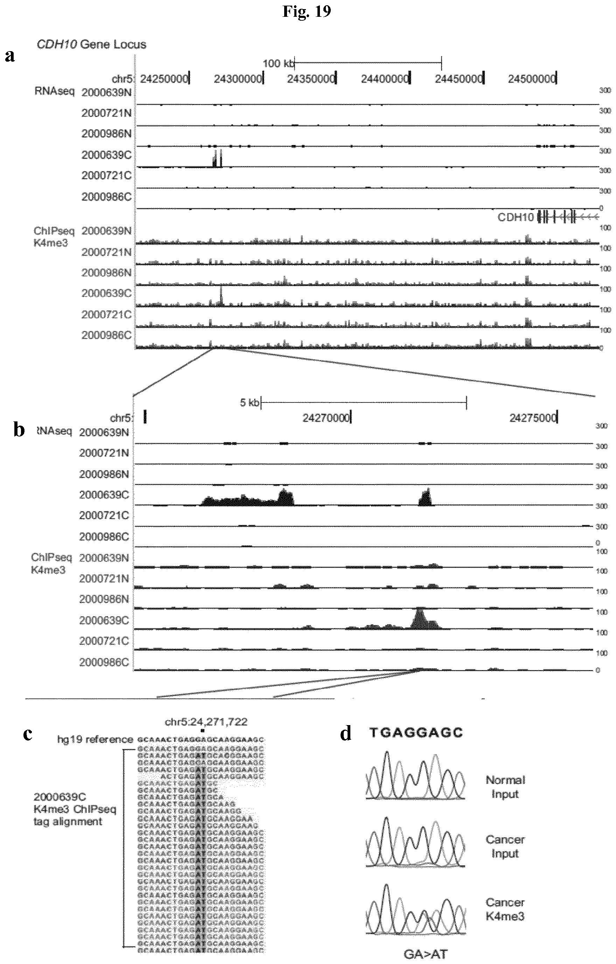

**Figure 19****. *CDH10* Locus Somatic Mutation Analysis.** (a) Genome browser view of *CDH10* locus. (b) Magnified view. (c) H3K4me3 ChIPseq tag alignment, showing variant and allele bias. Note that the mutation is a dinucleotide substitution compared to the hg19 reference. (d) Sanger sequencing traces of normal and cancer input DNA and H3K4me3 ChIP DNA. Cancer input contains a small fraction of the mutant allele, and the mutant allele is enriched by H3K4me3 ChIP.

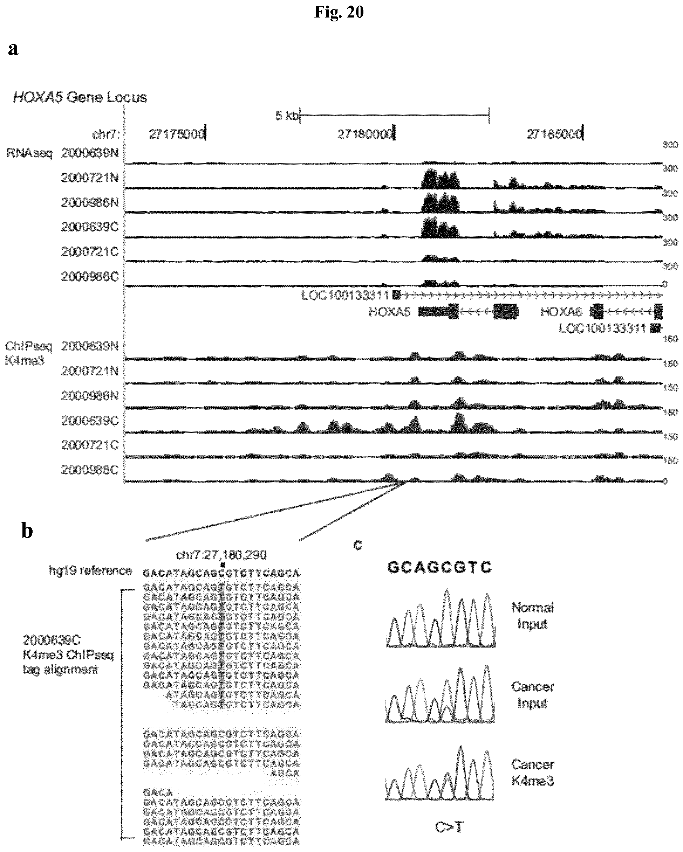

**Figure 20****. *HOXA5* Locus Somatic Mutation Analysis.** (a) Genome browser view of *HOXA5* locus. (b) K4me3 ChIPseq tag alignment, showing variant and allele bias. (c) Sanger sequencing traces of normal and cancer input DNA and H3K4me3 ChIP DNA. Cancer input contains a small fraction of mutant allele, and the mutant allele is enriched by H3K4me3 ChIP.

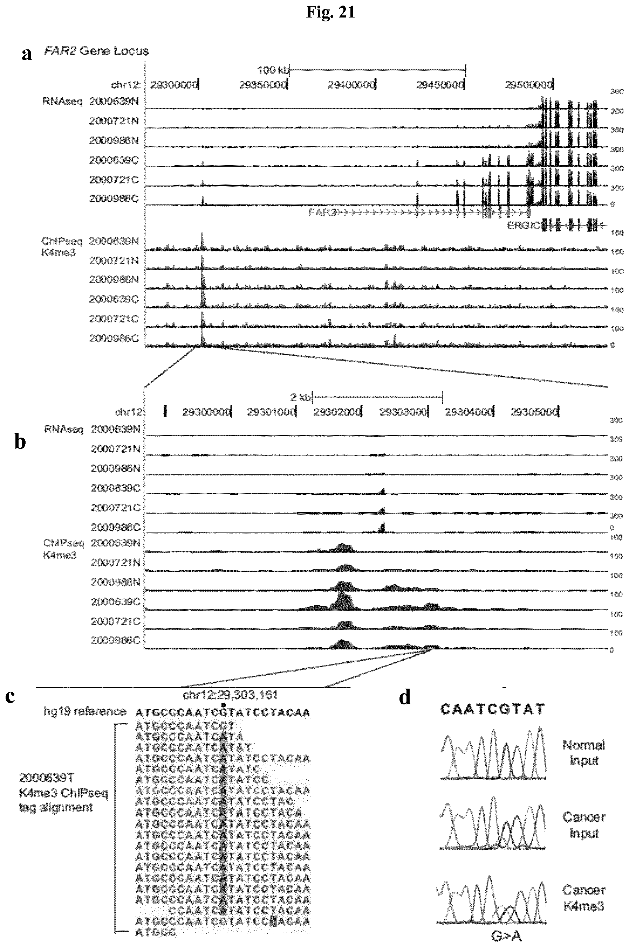

**Figure 21****. *FAR2* Locus Somatic Mutation Analysis.** (a) Genome browser view of *FAR2* locus. (b) Magnified view. (c) H3K4me3 ChIPseq tag alignment, showing variant and allele bias. (d) Sanger sequencing traces of normal and cancer input DNA and H3K4me3 ChIP DNA. Cancer input contains a small fraction of mutant allele, and the mutant allele is enriched by H3K4me3 ChIP.

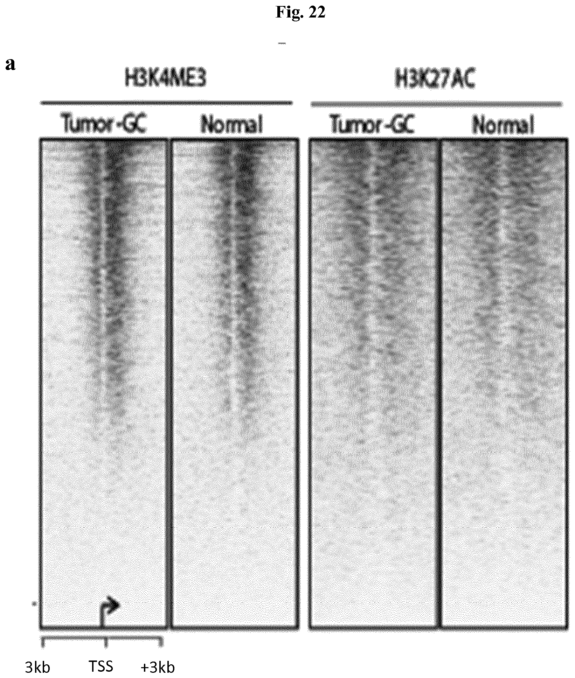

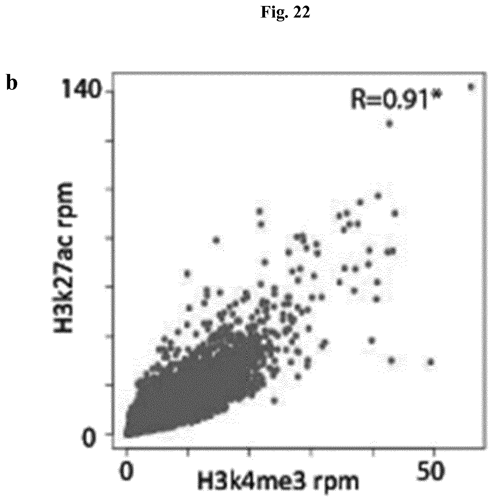

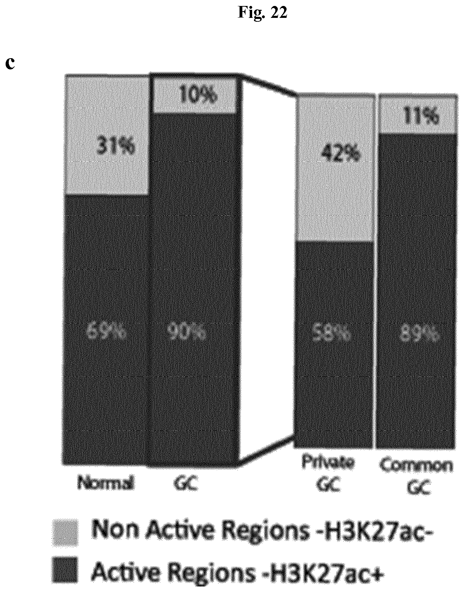

**Figure 22****. Characterization of H3K4me3 identified regions.** (a) H3K4me3 and H3K27ac showing standard bimodal distribution enriched around the TSSs. (b) H3K4me3 showing a strong positive correlation with H3K27ac in tumors (r=0.91,p<0.001) (c) Proportion of active (i.e. H3K27ac positive) regions in, common (i.e. recurrent) GC promoters was higher than private promoters, as well as promoters in normal samples.

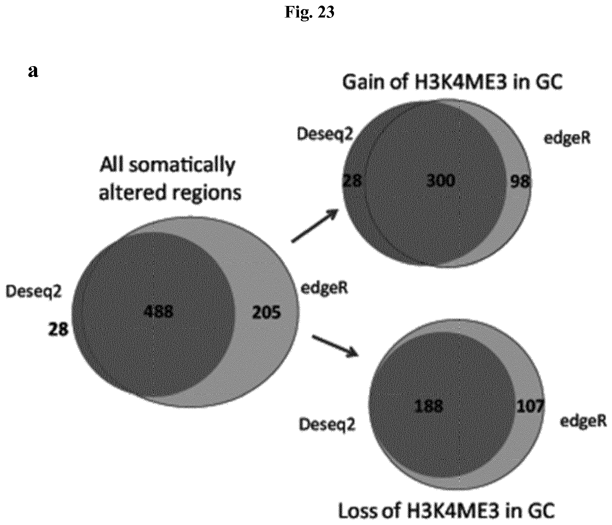

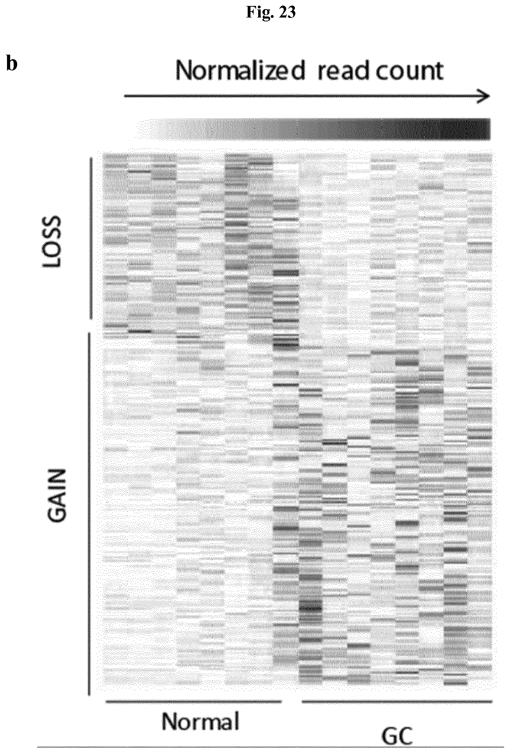

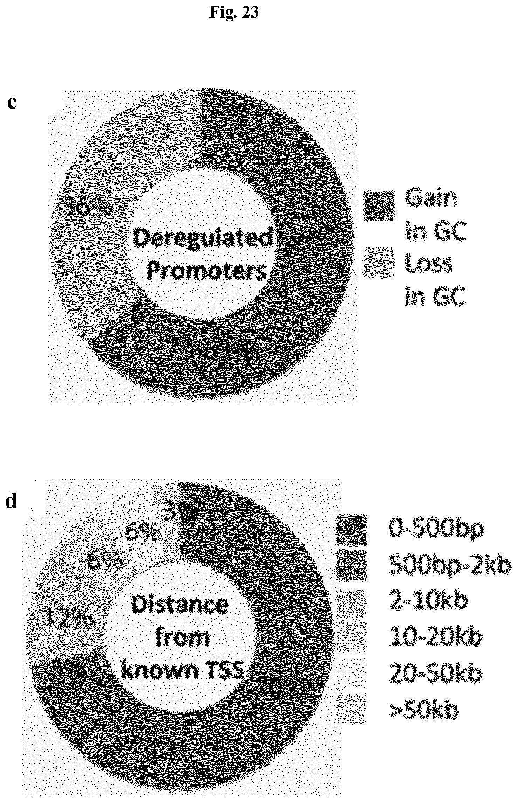

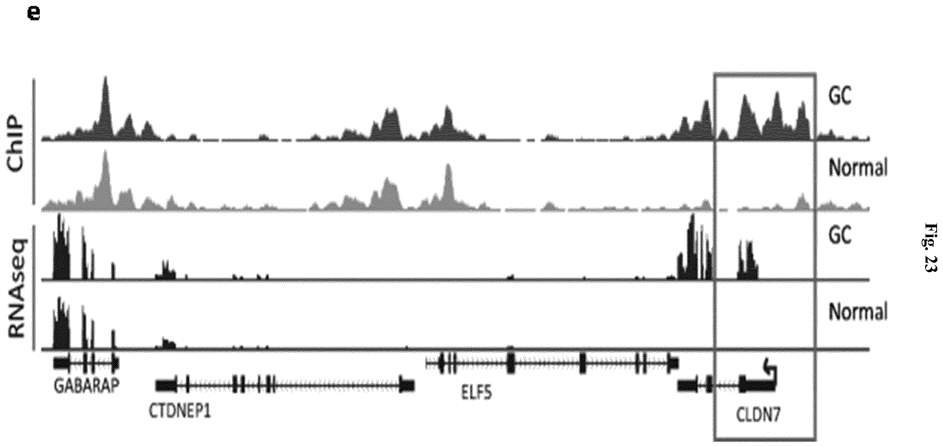

**Figure 23****. Somatically altered H3K4me3 regions in 8 GCs vs. normal tissue.** (a) DESeq2 identified 516 differential regions between GC and normals and similar results were obtained on analysis with edgeR. (b) Heatmap showing distinct separation between H3K4me3 signals of GC and normal tissues in the 516 differential regions. (c) Donut chart showing that 63% of the regions gained H3K4me3 in GC. (d) Donut chart of distribution of distances between differential regions and closest TSS e) Bedgraph track for GC associated gene CLDN7 showing enrichment of H3K4me3 in GC with concordant gain in RNAseq at its TSS compared to normal tissue.

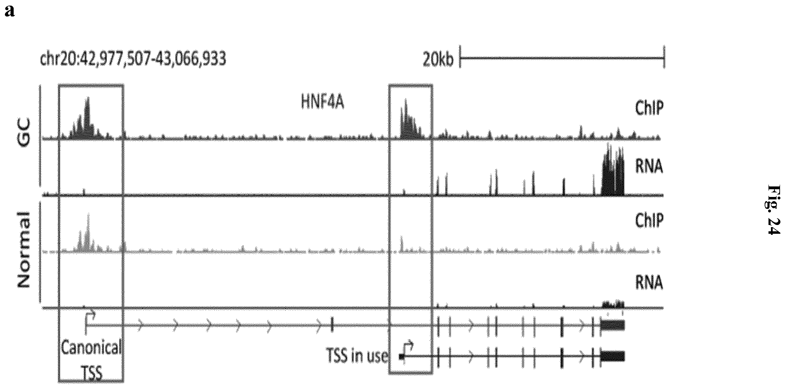

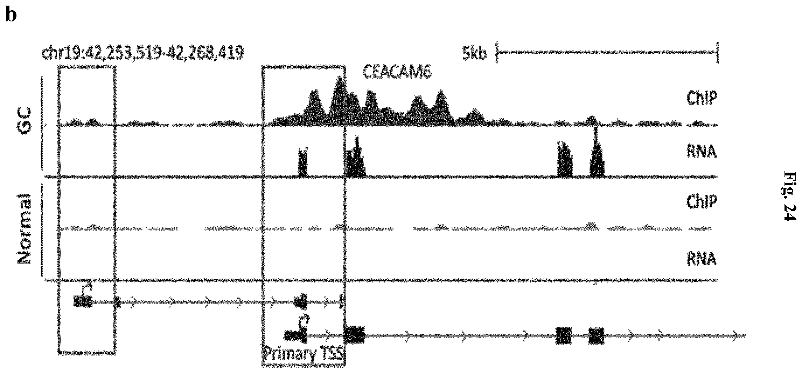

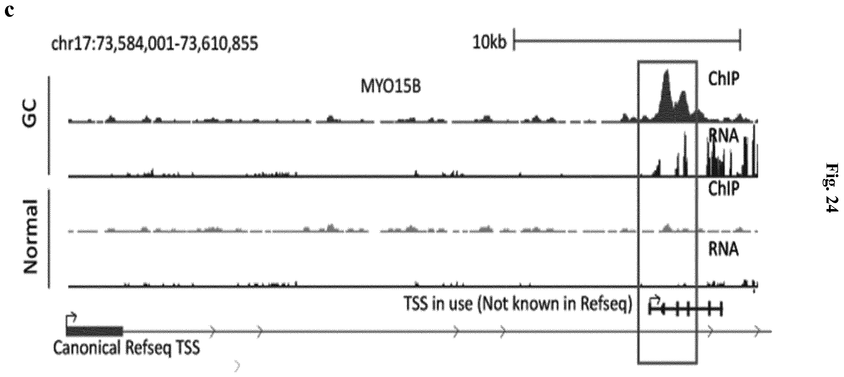

**Figure 24****. Alternative promoter usage at differential H3K4me3 loci in GC.** (a) Gain of H3K4me3 at a shorter known isoform of HNF4A while the canonical longer isoform has equal intensity peaks for H3K4me3. (b) Presence as well as gain of H3K4me3 only at one primary isoform of CEACAM6. (c) Gain of H3K4me3 in GC at an unsupported transcript of MYO15B not known in Refseq, and also supported by RNAseq expression in GC.

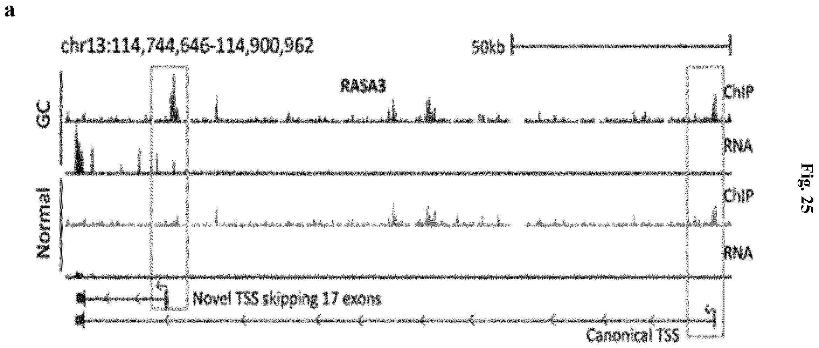

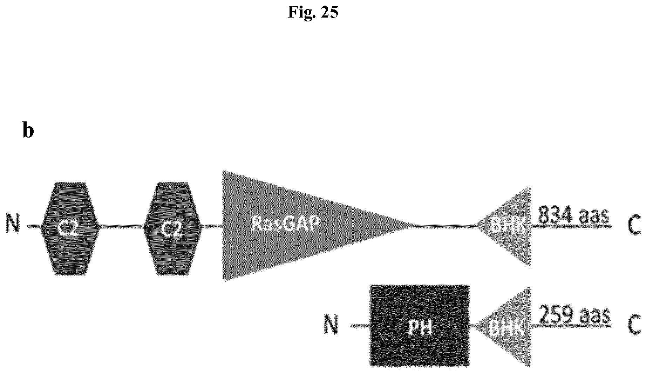

**Figure 25****. Example of novel 5' start site in RASA3.** a) Region of H3K4me3 gain in GC was observed almost 127kb downstream of known TSS marking site of novel transcription initiation. RNAseq assembly supported the presence of a new isoform in GC. b) Impact on protein domain of RASA3 on skipping 17 exons and forming a shorter isoform. The shorter isoform loses the RasGAP domain regulating the activity of RAS, instead has a predicted Pleckstrin homology domain.

### DETAILED DESCRIPTION OF THE PRESENT INVENTION

The present invention refers to a method for determining the activity of at least one promoter in a cancerous biological sample relative to a non-cancerous biological sample, as specified in claim 1. The method comprises mapping an isolated nucleic acid comprising at least one promoter sequence obtained from said cancerous biological sample against a reference nucleic acid obtained from said non-cancerous biological sample to obtain a read per kilo-base per million (RPKM) value or fragments per kilo-base per million (FPKM) value for said at least one promoter; and determining the differential activity of the at least one promoter sequence in the nucleic acid relative to the activity of the at least one promoter in the reference nucleic acid sequence using said RPKM or FPKM value, wherein the step of determining the differential activity of the at least one promoter sequence comprises determining that the RKPM or FPKM value for the at least one promoter in the nucleic acid obtained from the cancerous biological sample is: (i) greater than between a 1 to 20-fold, such as a 1-fold, 2-fold, 3-fold, 4-fold or 5-fold, change in mean RPKM or FPKM value relative to the RPKM or FPKM value of the at least one promoter in the reference nucleic acid obtained from the non-cancerous biological sample; and (ii) greater than a 0.1 RPKM or FPKM range relative to the RPKM or FPKM value of the at least one promoter in the reference nucleic acid obtained from the non-cancerous biological sample; wherein the cancer is gastric cancer; and wherein the nucleic acid is isolated from said cancerous biological sample by immunoprecipitation of chromatin, wherein said nucleic acid comprises said at least one promoter, the immunoprecipitation being achieved by an antibody specific for a modified histone protein comprising at least one histone modification selected from the group consisting of H3K4me3, H3K4me1, and H3K27ac.

The cancerous and non-cancerous biological sample described herein may comprise a single cell, multiple cells, fragments of cells, body fluid or tissue. In some embodiments the cancerous and non-cancerous biological sample may be obtained from the same subject or, alternatively, a different subject.

The nucleic acid may be isolated from said cancerous biological sample by immunoprecipitation of chromatin, wherein the nucleic acid comprises at least one promoter.

In some embodiments the antibody is specific to at least one histone modification selected from the group consisting of H3K4me3, H3K4me1 and H3K27ac.

The isolated nucleic acid comprising at least one promoter may be amplified with at least one primer. In some embodiments, the amplified nucleic acid is used to construct a nucleic acid sequence library with said amplified nucleic acid.

In some embodiments the mapping step comprises calculating the RPKM values based upon the total sequence tags for the at least one promoter in the mapped nucleic acid relative to the reference nucleic acid.

In some other embodiments, the mapping step comprises calculating the FPKM values based upon identified transcript sequences associated with the at least one promoter in the mapped nucleic acid relative to the reference nucleic acid.

The at least one promoter may comprise an increase of SUZ12 binding sites relative to the total promoter population. In some embodiments, the at least one promoter is positioned adjacent to a gene associated with cell-type specification, embryonic development or transcription factors.

In another embodiment, the at least one promoter is positioned adjacent to a gene associated with cancer. The gene may be selected from NKX6-3, SALL4, HOXB9, MET, TNK2, KLK1, FAR2, HOXA11 or HOXA11-AS. The cancer may be gastric cancer.

In another embodiment, the at least one promoter may comprise a cryptic promoter.

In some embodiments, the method further comprises: generating a matrix of sequencing tag counts for said at least one promoter based on the mapping step; and analyzing said matrix of sequencing tag counts prior to determining the differential activity the at least one promoter sequence in the nucleic acid.

In one embodiment, the generating step of the above method comprises calculating the matrix based upon a sequence tag count for the at least one promoter in the mapped nucleic acid relative to the reference nucleic acid.

In one embodiment, the analysis step of the above method comprises analyzing the matrix using a DESeq2 algorithm. The DESeq2 algorithm is a genomic analysis tool known in the art for differential analysis of count data, using shrinkage estimation for dispersions and fold changes to improve stability and interpretability of estimates. DESeq2 enables a quantitative analysis focused on the strength rather than the mere presence of differential expression. In particular, DESeq2 provides methods to test for differential expression by use of negative binomial generalized linear models; the estimates of dispersion and logarithmic fold changes incorporate data-driven prior distributions.

In one embodiment, the at least one promoter is positioned adjacent to a gene associated with cancer.

In one embodiment, the gene is RASA3, GRIN2D, TNNI3, SHD, ATP10B, SMTN, MYO15B, C2orf61, LINC00443 or ACHE.

In one embodiment, the differential enrichment is identified based upon a FDR rate of 10% and an absolute fold change of 1.5.

In one embodiment, the differential activity is identified based upon a FDR rate of 10% and an absolute fold change of greater than 1.5.

### EXPERIMENTAL SECTION

### Example 1

### Methods

### Tissue samples

Primary patient samples were obtained from the Singhealth tissue repository, and collected with approvals from institutional research ethics review committees and signed patient informed consent.

'Normal' (non-malignant) samples used in this study refer to samples harvested from the stomach, from sites distant from the tumor and exhibiting no visible evidence of tumor or intestinal metaplasia/dysplasia upon surgical assessment. Tumor samples were confirmed by cryo-sectioning to contain >40% tumor cells.

### Nano-ChIPseq

Nano-ChlPseq was performed as previously described, with the addition of a tissue dissociation step. Fresh-frozen cancer and normal tissues were dissected using a razor blade in liquid nitrogen to obtain ∼5mg sized pieces (∼ 5 µl by apparent volume). Tissue pieces were fixed in 1% formaldehyde/TBSE buffer for 10 minutes (min) at room temperature. Fixation was stopped by addition of glycine to a final concentration of 125 mM.

Tissue pieces were washed 3 times with TBSE buffer, and transferred into Lysonator cartridges (SG Microlab Devices, Singapore). Tissues were dissociated following the manufacturer's guidelines (4K Hz for 3 min), and taken directly to the lysis step in the Nano ChIP assay. Dissociated tissues were lysed in 200 µl of lysis buffer and divided into two 1.5 ml tubes for sonication (6 min) using a Bioruptor (Diagenode). For each tissue, ChIPs were performed using the following antibodies: H3K4me3 (07-473, Millipore); H3K4me1 (ab8895, Abcam); H3K27ac (ab4729, Abcam); H3K36me3 (ab9050, Abcam); H3K27me3 (07-449, Millipore), using same chromatin preparation.

After recovery of ChIP and input DNA, whole-genome-amplification was performed using the WGA4 kit (Sigma-Aldrich) and Bpml-WGA primers.

Amplified DNA was digested with Bpml (New EngliandBiolabs), ligated to a 2nd Bpml adaptor and digested again to trim the WGA primer regions and semi-random priming ends. 15 ng of amplified DNA was used for each Illumina sequencing library using the ChIPseq kit (Illumina). Each library was sequenced on one lane of HiSeq2000 to obtain either 36- or 101-base single reads.

### Nano-ChIPseq Read Mapping and Peak Calling

Sequencing tags were mapped against the human reference genome (hg19) using Burrows-Wheeler Aligner (BWA) software (version 0.7.0) and the "aln" algorithm. 101-base reads were trimmed by the first and last 10-base to increase SNP call performance. Uniquely mapped tags were used for peak calling by CCAT version 3.0. Peak regions were filtered by a fold-above input cut-off of 8 for H3K4me3, H3K27ac, 5 for H3K4me3 and H3K36me3 and 1.5 for H3K27me3 marks. For H3K4me3 and H3K4me1 histone modifications, peak regions from all tissue samples were pooled, and overlapping peak regions were merged to create a total set of peak regions for that modification for promoter and enhancer analysis. Normal input vs cancer input CCAT3 region sets with the same fold-cut-off were used to remove potential amplified regions for H3K4me3 and H3K4me1 regions. To quantify peak heights, we analyzed the ChIPseq data using Cufflinks (version 2.0.2). RPKM values were estimated for H3K4me3 and H3K27ac for promoter regions, H3K4me1 and H3K27ac for enhancer regions. Batch effects were assessed using principal components analysis (PCA) using the '*prcomp*' function in R (version 2.15), and adjusted using ComBat3 after log transformation of RPKM values from Cufflinks. 3D-PCA plots were plotted using the '*rgl*' package in R (version 2.15).

### Identification of Somatically Altered Regulatory Elements

Somatically altered promoter and enhancer sets were identified using two methods - a "threshold" method and a linear model approach. The final set of altered elements was generated by combining the results from both methods.

### "Threshold" method

H3K27ac ChIPseq ComBat-adjusted FPKM values for all promoters (H3K4me3-marked) and enhancers (H3K4me1-marked, but not overlapping with H3K4me3 peak regions) were filtered by (i) greater than 2-fold (absolute) change and (ii) greater than 0.5 (absolute) difference in mean values between 5 tumour and 5 normal samples. This was also performed for the H3K4me3 and H3K4me1 ChIPseq data. Altered elements were identified from the union of regions obtained for H3K27ac and H3K4me3 analyses (promoters), or H3K27ac and H3K4me1 (enhancers).

### Linear model method

Box plots were plotted for the log-transformed ChIPseq data to assess the normality assumption, prior to applying an empirical Bayes linear model approach to obtain differentially altered regions between the tumor and normal samples (Fig. 5). After the model was fitted, assessment was also made to ensure pvalue distributions were reasonable (Fig. 5). To obtain altered promoters and enhancers, a p=0.05 threshold level of significance was taken.

### RNAseq

RNAseq libraries were prepared using the Illumina Tru-Seq RNA Sample Preparation v2 protocol, according to the manufacturer's instructions. Briefly, poly-A RNAs were recovered from 1µg of total RNA using poly-T oligo attached magnetic beads. The recovered poly-A RNA was chemically fragmented and converted to cDNA using SuperScript II and random primers.

The second strand was synthesized using the Second Strand Master Mix provided in the kit followed by purification with AMPure XP beads. The ends of the cDNA were repaired using 3' to 5' exonuclease activity. A single adenosine was added to the 3' end, and the adaptors were attached to the ends of the cDNA using T4 DNA ligase.

The fragments with adapters ligated onto both ends were enriched by PCR. Libraries were validated with an Agilent Bioanalyzer (Agilent Technologies, Palo Alto, CA). Libraries were diluted to 11 pM and applied to an Illumina flow cell using the Illumina Cluster Station. Sequencing was performed on an Illumina High Seq2000 sequencer at the Duke-NUS Genome Biology Facility, with the paired-end 76-bp read option.

### RNAseq analysis

Reads were aligned to the human reference genome using TopHat v1.25. Unmapped reads were then aligned to potential splice junctions that were either: (i) present in Ensembl 60 transcript annotations, or (ii) suggested by "expression islands", i.e. clusters of reads from transcripts that were not present in the annotations. Transcript abundances by FPKM value were estimated using Cufflinks (version 1.0.0) without using reference transcripts. *De novo* assembled transcripts from the tumor/normal pairs were filtered against the RefSeq transcript database to identify non-RefSeq annotated regions.

### RefSeq TSS Overlap Analysis

RefSeq transcripts were downloaded from the UCSC browser, and RefSeq annotated TSSs were defined by extending transcript start positions by -/+500 bases. Somatically altered H3K4me3 peak regions were compared against RefSeq TSS regions to determine overlaps. H3K4me3 regions with no overlap with RefSeq TSSs (-/+500 bases) were deemed non-RefSeq promoters (aka cryptic promoters).

*De novo* assembly of RNAseq reads was performed by Cufflinks (version 1.0.0) without the reference transcript set. Non-RefSeq transcripts were defined by filtering the Cufflinks *de novo* exon output against the RefSeq exons (minimum 1-base overlap). This non-RefSeq transcript set was intersected against the cancer-associated H3K4me3 regions (minimum 1-base overlap).

### Quantitative RT-PCR

Quantitative PCR was performed using a SYBR Green PCR kit (Life technologies, USA). GAPDH was used as a control gene for normalization. All PCR reactions were performed in triplicate.

### 5' Rapid Amplification of cDNA Ends (5' RACE)

5' RACE was performed using the 5' RACE System for Rapid Amplification of cDNA Ends (version 2) kit (Invitrogen). 1µg of total RNA was used for each reverse transcription reaction with the Moloney Murine Leukemia Virus (M-MLV) reverse transcriptase, and gene specific primers for *MET* Refseq exon 3 (5'CTTCAGTGCAGGG3') or *NKX6-3* Refseq exon 1 (5'GAAGGTAGGCTCCTC3').

RNase H and RNase T1 were used to degrade the RNA, followed by the purification of first strand cDNAs with S.N.A.P. columns. Homopolymeric tailing of cDNAs were then used to create abridged anchor primer binding sites. Amplification of first strand cDNAs was performed using SuperTaq Plus Polymerase (Applied Biosystems) for 5'RACE outer PCR with the abridged anchor primer, and gene specific primers for *MET* exon 3 (5'GGCTCCAGGGTCTTCACCTCCA3') and *NKX6-3* exon 1 (5'CCAGGCTGAGCACCGAGAAGGC3'). Subsequently, 5'RACE inner nested PCR was performed with the abridged universal amplification primer (AUAP), and the gene specific primers for *MET* exon 3 (same as outer 5' PCR) and *NKX6-3* exon 1 (5'GCTTGCGCAGCAGCAGGCGGAT3').

Gel electrophoresis was performed, and PCR bands of interest were excised for cloning with a TOPO TA Cloning Kit with pCR 4-TOPO vectors (Invitrogen). A minimum of five independent colonies were isolated, and the purified plasmid DNA were sequenced bidirectionally on an ABI 3730 automated sequencer (Applied Biosystems.).

### Microarray Analysis

200 GC and 100 matched normal gastric samples profiled on Affymetrix Human Genome U133 Plus 2.0 Genechip arrays were analyzed (GSE 15459). Data pre-processing was carried out using the *'affyPLM'* R package (v 2.15). Outliers were excluded, giving a total of 185 GC and 89normalsamples available for downstream analyses. Differential expression analysis between GC samples was performed using the *'limma'* R package (v 2.15). Genes with false discovery rates (FDR) < 0.05 were considered to be differentially expressed. Genes used for differential expression analysis were those emerging from the GREAT (v 2.02) analysis performed on the list of non-Refseq transcripts from RNAseq analysis. For survival analysis, the GC samples were clustered using a K-medoids approach aimed at finding K that minimizes the silhouette width. To assess correlation of different GC groups with clinico-pathological factors, a mosaic plot was plotted for categorical variables while a linear regression approach was employed for continuous variables. Significance (p < 0.05) of the correlation was determined by a Pearson chi-square test or a t-test accordingly. Kaplan-Meier survival analysis was employed with overall survival as the outcome metric. The log-rank test was used to assess the significance of the Kaplan-Meier analysis. Univariate and multivariate analyses were performed using Cox regression.

### Transcription Factor Binding Site (TFBS) Analysis (ENCODE)

ENCODE ChIPseq TFBS datasets (Txn Fac ChIP V3 - Transcription Factor ChIP-seq Clusters V3, 161 targets, 189 antibodies) were obtained from the UCSC browser. Overlaps against cancer-associated promoters and enhancers (or all promoters and enhancers) were counted for each TF. TF site counts were divided by the base coverage length of each corresponding promoter, enhancer, or total set to calculate the TF site frequency per 10 kb coverage.

### DNA Methylation Profiling

Ilumina Human Methylation450 (HM450) Infinium DNA methylation arrays were used to assay DNA methylation levels between the gastric tumor/normal pairs. Methylation _-values were calculated and background corrected using the *methylumi* package in Rpackage version 2.4.0. Normalization was performed using the BMIQ method (wateRmelon package in R).

Probes containing SNPs and repeats were removed. Additionally, probes on the X and Y chromosomes were also removed. Control groups used included all 21,692 promoter regions. For each group (control, gain, and loss), we identified HM450probes overlapping with the promoter regions (135606, 2268, 963 probes for all, cancer-gained, and cancer-lost respectively). Probes with a detection p-value >0.05 were excluded. Probes that had an average change in DNA methylation, between the tumor and normal pairs, of at least 0.2 _ (in either direction) were selected and plotted. A two-sample Wilcoxon test was performed.

### Single-nucleotide Variation (SNV) Detection

The sequencing data were pre-processed according to the best practice workflow in Genome Analysis Toolkit (GATK version 2.6). Specifically, samtools was used to remove PCR duplicates. The remaining sequences were corrected for misalignments due to the presence of indels followed by base quality score recalibration. Single nucleotide variants (SNVs) in each GC/normal pair were called using MuTect11. We used SNV attributes reported by MuTect to classify the SNVs as either dbSNP sites or private SNVs. The dbSNP sites have the following criteria: (i) it is a known dbSNP site, (ii) the site is powered to detect a mutation (a.k.a covered site), and (iii) it passes the variant filters implemented in MuTect.

### Detection of Allele Bias and Private/Somatic Mutations

The alternate allele fraction was determined at each site by computing alternate allele frequencies.

Homozygous dbSNP sites showing an average alternate allele fraction greater than 0.9 in GC/normal pair were excluded. We focused on heterozygous sites showing an alternate allele fraction difference greater than 0.3 in a GC/normal pair (ie allele bias).

Allele-biased sites mapping to regions exhibiting cancer-associated chromatin mark alterations were assessed for functional impact using RegulomeDB12. For RegulomeDB hits, we also confirmed by quantitative pyrosequencing a lack of allele bias in input DNA populations. For somatic mutations, the focus was on private (non-dbSNP) SNPs. These "private" SNPs were identified using the following criteria: (i) it is a novel non-dbSNP variant, (ii) the alternate allele fraction in tumor is greater than 0.3 at a covered site, or 0.5 at uncovered sites, (iii) the site coverage has at least 14 reads in GC, (iv) there is no mutant allele at the uncovered site in normal tissue. Besides MuTect, private SNPs were also considered and identified using CLC Genomics Workbench (CLC Bio). Private SNPs mapping to regions exhibiting cancer-associated chromatin mark alterations were regarded as candidate somatic mutations.

### Quantitative Pyrosequencing, TF Site Prediction

Pyrosequencing was performed on a PyroMark Q24 (Qiagen). Results were analyzed with PyroMark software for allele quantification. For ChIP-qPCR-pyrosequencing, PCR primers were used for both real-time PCR quantification of ChIP DNA and allele-quantification by pyro-sequencing with WGA-amplified DNAs as a template. Quantification results and allele representations were combined to estimate the fraction of two alleles in the ChIP signal. Binding site predictions were performed using the TFBIND13 (http://tfbind.hgc.jp/).

### Luciferase Assay

Luciferase reporter assays were performed using Promega pGL3 (firefly luciferase) and RLSV40 (Renilla luciferase) plasmids. The *FOS* gene promoter was amplified by PCR from human genomic DNA with BglII-HindIII linker primer, and ligated into the pGL3-BASIC plasmid. *HOXA11*-associated fragments (∼350 bp) containing either wild-type or mutated alleles were amplified from ChIP-WGA DNA with BglII linker primers, and cloned upstream of the *FOS* promoter. Insert directions and allele identities were confirmed by Sanger sequencing. KATO-III GC cells were seeded at 1x106 cells per 24-well plate, transfected with the pGL3 reporter or derivatives (100ng per well), and pRLSV40 (20ng per well) using Lipofectamine 2000 (Invitrogen). Cells were harvested 42 hours post transfection, lysed in PLB buffer provided by the Dual-Luciferase Kit (Promega) and luciferase activity was measured. Reading of firefly luciferase activity was divided by *Renilla* luciferase activity to normalize transfection efficiencies.

### Discussion

Nano-ChlPseq has been validated down to the 1,000-cell scale (Fig. 6). Five matched pairs of primary GCs and normal gastric samples were profiled (Fig. 1a for clinical details). The chromatin marks included i) tri-methylated histone H3 lysine 36 (H3K36me3), associated with transcribed regions; ii) tri-methylated histone H3 lysine 27 (H3K27me3), repressed regions; and iii) histone H3 H3K4me3, H3K4me1, and H3K27ac (ac=acetylation), marking active promoters and enhancers. For each mark, >45 million uniquely mapped Illumina sequencing tags were generated and called peak regions using CCAT (Fig. 7). Genomic regions in tumor samples exhibiting an abnormal abundance of ChIP input tags, likely reflecting genomic amplification were excluded from downstream analyses (Fig. 8). Genome-wide chromatin profiles for both normal and cancer tissues were successfully obtained (Fig. 1b), despite limited material (∼5mg tissue for all marks). For example, cancer-specific gain of promoter activity (increased H3K4me3 and H3K27ac) at the *CDX2* gene, associated with intestinal metaplasia was observed (Fig. 1c).

Comparison of the chromatin marks revealed that regions of active transcription (H3K36me3) were exclusive to regions of repressive chromatin (H3K27me3) (Fig. 1b, d, GC 2000721; see Fig. 9). To define potential promoter (marked by H3K4me3) and enhancer regions (H3K4me1 positive but H3K4me3 negative), H3K4me3 and H3K4me1 peak regions from the 5 tissue pairs were intersected (Fig. 1e). >21K promoter and >125K enhancer regions were identified. 64% of promoters were active (H3K27ac-positive) and 19% of putative enhancers were marked by H3K27ac in at least in one tissue (Fig. 1e).

To identify somatically altered promoters and enhancers in GC, sequencing tag densities were quantified and compared (read per kilo-base per million tags, RPKM) between GCs and normal tissues (Fig. 1b, 10, 11). 639 promoters exhibiting differential H3K4me3 and H3K27ac modifications between GCs and normal tissues, and 975 somatically altered enhancers were identified (see Methods). Principal components analysis (PCA) and clustering analysis using these somatically altered elements confirmed a separation between GCs and matched normal tissues (Fig. 1f-i, 11).

Gains of new promoters in primary GCs outweighed promoter losses (472 gained vs. 167 lost, Fig. 2a). Unexpectedly, the majority of gained promoters (58%) localized to regions >500 base-pairs (bp) from annotated transcription start sites (TSSs) found in Refseq, a reference database of transcript sequences. The fraction of "cryptic promoters" in gained promoters was significantly greater than either the global promoter population or promoters lost in GC (∼44%, p<7.1x10-6, Fisher's Exact Test). To ask if cancer-associated promoters (including cryptic promoters) were associated with *bona fide* RNA transcripts, RNA sequencing (RNAseq) was then performed on 12 tumor/normal pairs, including the index 5 GCs. The majority of promoters (59.5%, 380 promoters) were associated with detectible RNA transcripts (Fig. 2a). 192 transcripts exhibiting >4-fold expression changes in GCs compared to normal tissues were identified, and almost half of these (48%, 92 promoters) were due to cryptic promoters, supporting their cancer-specific nature (Fig.2b). Using targeted qPCR, 10 cryptic promoter-driven transcripts were experimentally validated (Fig. 12).

Genes located near cancer-associated promoters were significantly enriched in gene sets related to gastrointestinal neoplasms/digestive system cancers (Fig. 2c; p<1x10-5 by GREAT analysis). It was discovered that cryptic promoters frequently drove expression of these nearby genes, through non-canonical mRNA isoforms with altered 5' structures. For example, GC 2000721 exhibited tumor specific expression of the *MET* receptor via an internal cryptic promoter (Fig. 2d-f, 13), producing a truncated isoform lacking the N-terminal Sema domain which regulates receptor dimerization and signaling. 5' RACE (Rapid Amplification of cDNA Ends) analysis of *MET*-expressing Hs746T GC cells confirmed expression of this truncated *MET* isoform (Fig. 13). Interestingly, genes located near cancer-associated promoters also exhibited significant functional enrichments in transcription factor function, embryonic development, and cell-type specification (p<2×10-6, FDR q<1×10-3; Fig. 2c). For example, *NKX6-3,* a nervous system and stomach tissue developmental regulator, exhibited cancer specific expression via a new 5' exon skipping the canonical RefSeq *NKX6.3* 1^{st} exon (Fig. 2g, 14), resulting in a novel 184-amino-acid N-terminus modifying the homeobox domain (Fig. 14). RACE was used to confirmed expression of these new 5' exons in GC lines (Fig. 14). Similar altered 5' transcript structures were observed for the homeobox transcription factor *HOXB9* (Fig. 2h, 15). These results demonstrate cryptic promoter activation in GC. Non-canonical transcript isoforms generated by these promoters may also produce proteins with altered cellular functions.

To validate these expression patterns, it was confirmed that genes driven by H3K4me3-marked cancer associated promoters exhibited similar tumor up regulation in an expanded microarray cohort of 185 GCs and 89 normal gastric tissues (p=5.68x10⁻⁶; Fig. 2i, 16). GCs exhibiting high expression of H3K4me3-marked genes exhibited higher M-stages (p=0.033; Fig. 17), diffuse Lauren's histology (p = 9.99x10-5; Fig. 17), and worse overall survival compared to GCs where these genes were lowly expressed (Fig. 2j; log-rank test p-value=0.04). Multivariate Cox regression analysis revealed that the survival outcomes not independent of tumor stage (p=0.74). Genes driven by H3K4me3-marked promoters in GC may thus contribute to pathologic and clinical features of GC.

When mapped against genomic occupancy data of 161 transcription factors (ENCODE consortium), cancer-associated promoters exhibited a generalized depletion of previously-defined transcription factor binding sites (Fig. 3a, 18a), but a significant enrichment in SUZ12 and EZH2 binding (p=1.2×10⁻²⁴ and p=1.1×10⁻⁴ for SUZ12 and EZH2, Bonferroni corrected). SUZ12 and EZH2 are components of polycomb complex 2 (PRC2), which targets key developmental genes in embryonic stem cells (ESCs), and is also involved in cancer progression. In contrast, GC associated enhancers did not display SUZ12/EZH2 enrichment, but were associated with developmental regulators including forkhead (FOX), GATA family members, and the FOS and JUN cell-cycle regulators (p<0.05) (Fig. 3b).

Several commonalities occurred between GC-associated promoters and PRC2-targeted regions in ESCs. First, the SUZ12 sites overlapping with GC promoters were sourced from ESCs and embryonic carcinoma cells, and not other ENCODE cell types (for example, lymphoblastoid lines). Second, in ESCs, PRC2 binding is associated with bivalent/poised chromatin states (H3K4me3 and H3K27me3 positive), and similarly the GC promoters were enriched at regions exhibiting H3K4me3 and H3K27me3 bivalency in ESCs (p < 2.2x10⁻¹⁶; Fig. 3c) an example is shown for the homeobox gene *ONECUT2* (Fig. 3d). Third, in ESCs, PRC2-targeted regions exhibit DNA methylation loss. Using Illumina methylation arrays, it was confirmed that GC-associated promoters also exhibited decreased DNA methylation levels relative to the overall population (p=7.07x10⁻⁴⁸) (Fig. 3e). Cancer-associated promoters in GC may thus be molecularly analogous to PRC2-targeted regions in stem cells.

To identify single nucleotide variants (SNVs) in the Nano-ChlPseq data, an analytical pipeline was developed based on MuTect, a sensitive mutation/variant identification algorithm. 335,918 unique SNVs were identified in the combined H3K4me3, H3K4me1, H3K27ac and input data. Supporting the accuracy of the variant calling pipeline, 99.8% of the SNVs (335,247) corresponded to known SNPs (dbSNP137). Among the identified dbSNPs, approximately ∼251,800 were heterozygous in at least one sample.

It was found that heterozygous SNPs mapping to regulatory elements could divided into non-allele biased and allele biased sites. At non-biased sites, Nano-ChlPseq sequence reads exhibited an equal proportion of reference and variant alleles. For example, GC 2000639 exhibited a cancer-associated promoter at the *TNK2* gene locus (Fig. 4a, b). In normal DNA from this patient, this region was heterozygous for dbSNP rs7636635 (Fig. 4c), and similarly in the tumor the H3K4me3-enriched reads were contributed by an equal proportion of reads bearing both reference and rs7636635 alleles (Fig. 4c, d). In contrast, allele biased sites displayed Nano-ChlPseq reads skewed towards one allele. This was observed in a cancer associated promoter at the *NUDT4* locus (Fig. 4e, f). Analysis of normal DNA from this patient confirmed heterozygosity for rs4761701 (Fig. 4g), but in tumors the H3K4me3 enriched reads were primarily contributed by reads bearing the rs4761701 allele rather than the reference allele (Fig. 4g, h).

It was reasoned that the allele-biased sites in cancer samples might be caused by either loss-of heterozygozity (LOH), or active enrichment of particular alleles for chromatin marks (allele-specific regulatory elements). To identify the allele-specific regulatory elements associated with cancer, heterozygous sites exhibiting allele bias (SNP over-representation of >30%; Fig. 4i) were overlapped with regions exhibiting chromatin mark alterations between GCs and normal tissues. Of 151 candidate sites (Fig. 4j), 17 alleles (11%) of particular interested were focused on as predicted by RegulomeDB, a database of human regulatory variants, to influence protein DNA binding (RegulomeDB score 1 or 2) (Fig. 4k). 12 of these 17 sites could be further validated by quantitative pyrosequencing (5 sites could not be assayed due to PCR or sequencing failures), and of the remaining 12 the presence of allele bias in 9 sites (75%) within the ChIP-enriched reads but not in cancer and normal input DNAs was confirmed, indicating that this bias is not due to LOH in the cancer tissues. Four alleles corresponded to previously identified eQTLs (Fig. 4k), including the *KLK1* gene which showed allelic bias for the eQTL dbSNPrs2659104 (Fig. 4l-n). These results highlight a potential role for allele-specific regulatory elements in controlling patterns of GC gene expression, as several genes associated with the allele-biased sites have been previously implicated in GC (for example *CLDN4, MTAP, SERPINB5*).

Besides dbSNPs, also identified were private (non-dbSNP) SNVs overlapping with GC-associated regulatory elements. Four private SNVs were validated as *bona-fide* somatic mutations, being present in GCs but not normal tissues, occurring in non-coding regions associated with *CHD10, HOXA5, FAR2* and *HOXA11* (Fig. 5, 19-21). Among these, the *CHD10* and *FAR2* mutations exhibited allele bias in H3K4me3-enriched reads relative to input tumor DNAs, and also tumor-associated gene expression. *HOXA11*-associated A-T mutation was focused on, due to the involvement of *HOXA11* in numerous cancers. Five lines of evidence suggest that this somatic mutation is functional and not a bystander alteration. First, presence of this mutation in GC 2000639 was associated with H3K4me3 and H3K27ac promoter mark gain (Fig. 5a-c). Second, presence of this mutation was associated with upregulated *HOXA11* locus RNA expression in GCs (Fig. 5a). Third, in the H3K27ac sequence reads, the T mutant allele exhibited allele bias, being highly over-represented (96%) relative to input tumor DNA where its somatic mutation allele frequency is ∼10% or less (Fig. 5c-d).

Fourth, presence of this mutation is predicted to alter transcription factor binding (Fig. 5e). Fifth, in a luciferase reporter assay, genomic DNA fragments bearing the mutant T allele exhibited significantly greater transcriptional activity, compared to genomic DNA bearing the wild-type A allele (p=1.1x10-4, Fig. 5f). These results demonstrate that Nano-ChlPseq can identify functional regulatory somatic mutations in GC.

Regulatory elements are estimated to occupy 1.5-10% of the human genome, and strongly influence development and disease. However, locating these elements, and defining biological states regulating their activity, remains an important challenge. Here, Nano-ChIPseq was used to perform a first-pass survey of chromatin alterations in primary GCs. In future, Nano-ChlPseq could be expanded to other tumor types and to smaller cell numbers, facilitating analysis of diagnostic biopsies and drug resistant clones. From a translational perspective, our findings also suggest that cryptic promoters, and their associated non-canonical transcripts, could be conceivably exploited as biomarkers for cancer diagnostics.

### Example 2

### Methods

### Tissue samples

Primary patient samples were obtained from the Singhealth tissue repository, and collected with approvals from institutional research ethics review committees and signed patient informed consent.

'Normal' (non-malignant) samples used in this study refer to samples harvested from the stomach, from sites distant from the tumour and exhibiting no visible evidence of tumor or intestinal metaplasia/dysplasia upon surgical assessment. Tumor samples were confirmed by cryo-sectioning to contain >40% tumor cells.

### Nano-ChIPseq

Nano-ChIPseq was performed as previously described, with the addition of a tissue dissociation step. Fresh-frozen cancer and normal tissues were dissected using a razor blade in liquid nitrogen to obtain ∼5mg sized pieces (∼ 5 µl by apparent volume). Tissue pieces were fixed in 1% formaldehyde/PBS buffer for 10 minutes (min) at room temperature. Fixation was stopped by addition of glycine to a final concentration of 125 mM.

Tissue pieces were washed 3 times with TBSE buffer, and transferred into Lysonator cartridges (SG Microlab Devices, Singapore). Tissues were dissociated following the manufacturer's guidelines (4K Hz for 6 min), and taken directly to the lysis step in the Nano ChIP assay. Dissociated tissues were lysed in 200 µl of lysis buffer and divided into two 1.5 ml tubes for sonication (6 min) using a Bioruptor (Diagenode). For each tissue, ChIPs were performed using the following antibodies: H3K4me3 (07-473, Millipore); H3K4me1 (ab8895, Abcam); H3K27ac (ab4729, Abcam); H3K36me3 (ab9050, Abcam); H3K27me3 (07-449, Millipore), using same chromatin preparation.

After recovery of ChIP and input DNA, whole-genome-amplification was performed using the WGA4 kit (Sigma-Aldrich) and Bpml-WGA primers.

Amplified DNA was digested with Bpml (New England Biolabs). 10 ng of amplified DNA was used for each Illumina sequencing library. Library preparation was done using E6240 New England Biolabs kit and were then multiplexed before sequencing using the E7335 New England Biolabs kit.

### Nano-ChIP-seq read mapping and peak calling

Sequencing tags were mapped against the human reference genome (hg19) using Burrows-Wheeler Aligner software (version 0.7.0) and the 'aln' algorithm. MAPQ Filter of 20 was applied to remove low quality reads and all PCR duplicates were also removed using MarkDup from Picard. Uniquely mapped tags were used for peak calling by CCAT version 3.0, with fragment size 200bp and sliding window of 500bp with moving step of 50bp. for histone modifications. Peak regions were filtered by False Discovery Rate(FDR) 5%.

### ChipSeq Signal Analysis

H3K4me3 and H3K27ac signal intensity plots around the transcription start site (TSS) were plotted by calculating the average coverage of each chromatin mark around all annotated TSS in Refseq. A 6kb window around the known TSS was divided into bins of 100bp and coverage for both H3K4me3 and H3K27ac was calculated and was then averaged out across each bin.

All H3K4me3 regions were merged across GC samples and normal samples respectively, using bedtools and overlapping regions (1bp overlap) were counted as common regions. Regions without any overlaps were termed as Private regions. To provide a genomic null expectation for overlap between H3K4me3 regions among samples, consensus regions were shuffled over the entire reference genome, using shufflebed from bedtools, however excluding the ENCODE DAC Blacklisted regions and gap regions (These are a published set of regions from Dunham, I., et al. An integrated encyclopedia of DNA elements in the human genome. Nature 489, 57-74 (2012*)*). The regions were shuffled 10000 times and an empirical p value was generated using the overlap distribution.

### Somatically Altered Promoter Identification

H3K4me3 regions with differential enrichment in gastric cancer vs. normal samples were identified using the DESeq2 algorithm from Bioconductor. The enrichment is the gain of the H3k4me3 in the cancerous sample vs. the normal non-cancerous sample. A matrix of sequencing tag counts was generated by 1) combining all identified promoter regions across all GC and normal samples, and 2) determining the number of sequencing reads in each region across all samples. Both of steps 1) and 2) are carried out using bedtools.

DESeq2 tests determine the differential enrichment by use of negative binomial generalized linear models on various sequencing assays, including ChIPseq. The matrix of sequencing tag counts from all samples was generated as input for the DESeq2 tests by taking a union of H3k4me3regions identified across replicates using bedtools, and counting the no. of sequencing reads in each resulting promoter regions wherein the DESeq2 test fits a negative binomial generalized linear model to find promoter regions that are statistically different between Gastric cancer and normal samples ie. somatically altered promoters. Statistically different refers to a statistical threshold of a False Discovery Rate of 10% i.e q value 0.1 as well as an absolute fold change of 1.5.

Differential regions were identified for both tumor and normal groups as well as individual sample specific peaks.

### Alternate Promoter Identification

**GENCODE transcripts were downloaded from their ftp site while Refseq transcripts** were downloaded from the UCSC browser. Transcription support level information for GENCODE transcripts was downloaded from UCSC ftp site. Annotated TSSs were defined by extending transcript start positions by ±500 bases. Differentially enriched H3K4me3 peak regions were compared against the TSS regions to determine overlaps. *De novo* assembly of RNAseq reads was performed by Cufflinks-2.2.0.12 and unannotated H3K4me3 differential regions were filtered by overlap with the 1^{st} exons of the *de novo* assembly with class code 'j' or 'u'.

### RNAseq analysis

Reads were aligned to the human reference genome using TopHat 2-2.0.12 using unique mapping. The transcriptome was assembled *de-novo* using Cufflinks2-2.0.12 and all GC transcript assemblies were merged using 'cuffmerge-2.2.0' to get a consensus transcriptome. Raw RNAseq data for TCGA stomach adenocarcinoma was downloaded from the TCGA repository (http://cancergenome.nih.gov/).

### Poised Promoter Analysis

Differentially enriched regions were overlapped with CCAT3 called H3K27me3 peaks from 3 samples (1bp overlap) to determine its presence or absence.

### Discussion

### Identification of Promoter regions in GC

Using an expanded cohort of 8 primary GCs and matched normal samples, the promoter elements of gastric cancers (GC) were characterized with 2 promoter associated marks, H3K4me3 and H3K27ac, using Nano-ChIPseq. The peaks were called using CCAT3 and identified an average of 11k H3K4me3 and 34k H3K27ac peaks per sample. 70-80% of the H3K4me3 regions were common among samples in both GC and normal tissue, greater than expected by chance (p<0.001).

Both H3K4me3 and H3K27ac showed a standard bi-modal distribution enriched around transcription start sites (TSS) (Fig. 22a). H3K27ac showed a weaker signal compared to H3K4me3, which is expected given its widespread abundance marking all active regulatory regions. Greater than 99% of all H3K4me3 regions overlapped DNAsel hypersensitivity sites (from ENCODE). Both GCs (r=0.91, p < 0.001) (Fig. 22b) and normal samples (r=0.91, p <0.001) had strong positive correlations with H3K4me3 and H3K27ac. All H3K4me3 regions with presence of H3k27ac were marked as active promoter regions. The proportion of active (i.e. H3K27ac positive) regions in, common (i.e. recurrent) GC promoters was higher than private promoters, as well as promoters in normal samples (Figure 22c).

### Identification of somatically altered promoters in GC

To identify genome wide somatically altered promoters, the matrix of sequencing tag counts were compared between primary GCs and matched gastric normal tissue assuming a negative binomial distribution (as analyzed by the DESeq2 algorithm). Comparing all 8 GC samples to the pool of normal tissues, 516 robustly somatically altered regions (q<0.1, Fold change>1.5) were obtained, with -60% of them being gained or epigenetically activated in GC (Fig 23c).

Clustering of the H3K4me3 signals across samples for the identified regions confirmed a distinct separation (Fig. 23b). Similar results (95% concordance) were obtained using an alternative count based differential algorithm, ensuring our results were robust and method independent (Fig. 23a).

249 of the (48%) somatically altered regions were additional to the identified 639 promoters of Example 1 that used a 2 fold FPKM based sequencing tag density comparison. Gains of new promoters in primary GCs again outweighed promoter losses i.e. 148 (60%) gained vs. 101 lost. Overall, 620 promoter regions were gained in GC (70%) vs. 260 lost in GC.

Using a more comprehensive database of transcripts, the somatically altered regions were overlapped with 1kb windows around known GENCODE TSS to annotate them. 62% of the somatically altered promoter regions overlapped known transcripts. However, a substantial 38% lay beyond 500bp of annotated TSSs (Fig. 23d)

H3K4me3 enrichment at specific *loci* helped observe patterns of alternative promoter usage in GC influencing transcript selection. 553 (63% gained in GC) somatically altered promoter regions overlapped known transcripts. A preferential activation/repression was observed of one transcript over another in multi-transcript genes, such as HNF4A. HNF4A is a well-known transcription factor gene that regulates development of liver, kidney and intestines. In GC, HNF4A has been reported to be over expressed and a recent immunohistochemistry study showed its potential as a marker to distinguish GC tissues from breast cancer tissues.

H3K4me3 enrichment in GC (FC 2.52, q<0.001) was observed at a promoter almost 45kb downstream of the canonical HNF4A isoform TSS. The canonical promoter, on the other hand, showed equal lysine trimethylation in GC and normals, highlighting a GC specific usage of the downstream promoter and thus a shorter protein coding isoform of HNF4A (Fig 24a).

Other cancer related_genes with instances of such alternate promoter usage were EPCAM (FC 1.64, q<0.001), KRT7 (FC 2.00, q<0.001), AIM1L (FC 1.95, q<0.001), among others. The FC and q value statistics are derived from the DESeq2 analysis, where FC defines the fold change.

Somatically altered promoters also often overlapped only one transcript in genes associated with multiple transcripts, marking the primary promoter and a cancer-specific isoform (Fig 24b). CEACAM6 was a prominent example of this phenomenon, where only one isoform out of 2 known protein coding transcripts showed H3K4me3 enrichment in GC (FC 2.56, q<0.001). Multiple such instances was observed of usage of known alternate transcripts over canonical isoforms in GC determined by epigenetic activation or repression of their promoter regions by H3K4me3, such as CLDN4 (FC 2.71, q<0.001), SHD (FC 2.14, q<0.001), CEACAM18 (FC 2.10, q<0.01), and SULT2B1 (FC 2.33, q<0.001).

Instances of somatically altered regions overlapping GENCODE transcripts that had very poor transcriptional supporting evidence (tsl 2 or more) was also observed. Such GENCODE transcript annotations, with little or no mRNA support, are often not included in more curated databases such as Refseq.

109 enriched regions overlapping TSS of such transcripts was observed, supported by RNA expression in GC, which highlighted the GC specific usage of these otherwise, unsupported isoforms. One such example was MYO15B, a transcribed pseudogene that showed significant gain (FC 2.16, q<0.01) of H3K4me3 in GC at its unsupported isoform promoter while there was complete absence of H3K4me3 at its canonical isoform. (Fig 24c)

Additional cryptic promoters were identified marking novel 5' start sites of GC specific isoforms which were associated with *bona fide* RNA transcripts. A prominent example was Ras GTPase-activating protein 3 (RASA3), which showed differential H3K4me3 enrichment in GC samples at a promoter region almost 127kb downstream from the canonical transcript start site forming a much shorter novel isoform being transcribed only in GC tissues. The canonical isoform showed an equal amount of H3K4me3 in both GC and normal tissues. Other examples of such novel 5' start site isoforms were GRIN2D (FC 2.52, q<0.001), ONECUT3 (FC 2.52, q<0.001), and TNNI3 (FC 2.52, q<0.001).

Alternative promoter usage also changes in the protein composition of alternate GC specific isoforms. Using genomic sequence of known or *denovo* assembled isoforms arising from alternate promoters, the presence of protein domains was predicted and the domain composition compared to that of the canonical isoform to find instances of protein alterations.

Cases where the alternate isoform was supported by RNAseq were then selected for protein composition changes. 10 such high confidence genes were identified that showed protein domain diversity including RASA3 (Fig 25b, Table 1).

**Table 1**

| **Locus** | **Gene** | **Exon-Impact** | **Protein Impact Status** | **Function/Cancer Association** |
|---|---|---|---|---|
| chr13:114,769, 100-114,771,150 | RASA3 | Loss of 1st 17 exons | Loss of RASGap domain and predicted gain of Pleckstrin homology domain | Potential TSG in colorectal cancer. Stimulates GTPase activity of normal (not oncogenic) RAS p21 |
| chr19:48,900,300-48,903,900 | GRIN2D | Loss of 1st 2 exons | Loss of ANF receptor and and transmembrane domain. Gains 2 ligated ion channel binding sites | Glutamate receptor. Breast Cancer. |
| chr19:55,666,950-55,668,450 | TNNI3 | Loss of 1st 2 exons | Loss of troponin domain | Non-small-cell lung cancer. |
| chr19:4,283,950-4,285,850 | SHD | Loss of 1st 3 exons | Loss of SH2 domain. | - |
| chr5:160,108,900-160,112,700 | ATP10B | Loss of 1 st 4 exons | Loss of a transmembrane domain before ATPase domain | Phospholipid-translocating ATPase activity. |
| chr22:31,459,400-31,460,800 | SMTN | Gain of exon | Shifted position of Pfam domains | Muscle and actin binding. Breast cancer. Prostate cancer |
| chr17:73,606,350-73,609,450 | MYO15B | Loss of exon | Gain of SH3 and MyTH4 domain | Non protein coding |
| chr2:47,333,150-47,336,550 | C2orf61 | Loss of exon | Loss of 2 SHIPPO-rpt domains. | Colon cancer |
| chr13:107,302,100-107,305,850 | LINC00443 | Gain of exon. | Gain of ATP Synth C domain | - |
| chr7:100,490,950-100,495,550 | ACHE | Loss of exon | Addition of Co-esterase domain | Sporadic breast cancer. Gastroschisis |

The GC specific shorter isoform lacks the RasGAP domain that acts as a molecular switch downregulating the activity of Ras. In the absence of this domain, it could lead to increase in expression of GTP bound RAS and thus aberrant cellular proliferation.

Also observed was the presence of the H3K27me3 mark in somatically altered regions. In many cases, H3K27me3 mark was observed in either GC or normal tissues potentially marking transition of promoters from monovalent state to a bivalent poised state or vice-versa. For example, TNFSF9, a cytokine involved in tumor necrosis factor binding and shown to be expressed in Epstein Barr Virus (EBV) associated GC the showed gain of H3K4me3 and also presence of H3K27me3 in GC while the repressive trimethylation mark was absent in normal tissue. TNFS9 had concordant low levels of RNAseq expression (FPKM 4.9) with its poised epigenetic state in GC.

The identified somatically altered promoters highlight and confirm widespread alternate promoter usage in GC, as well as elucidating that alternate promoter usage can impact the protein domain composition of resulting proteins, that may be specific to GC.

As such, based upon the above observations and experimental data, using an expanded cohort of 8 primary Gastric Cancer (GC) in comparison to matched normal samples, the calculated read count matrix-based algorithm (Deseq2) was able to identify additional somatically altered promoter regions. The identification of somatically altered promoter regions highlight and confirm widespread alternate promoter usage specific to cancer, as exemplified with respect to gastric cancer, either through preferential alteration of the promoter of one transcript or by alteration of the promoter of the primary transcript in use in multi-transcript genes. Further, additional 'cryptic promoters' were identified in the expanded cohort marking 5' start sites of non-canonical isoforms.

The above non-canonical isoforms showed change in the domain composition of resulting proteins that may be specific to a particular cancer, for example in Table 1 illustrates the proteins that may be specific to GC. These cryptic promoters and associated non-canonical transcripts can be used as biomarkers for targeted therapy and cancer diagnostics. As such, the present invention and methods disclosed herein are advantageous in identifying and providing yet further biomarkers for the possible detection and diagnosis of cancer in a subject.

## Claims

1. A method for determining the presence or activity of at least one promoter in a cancerous biological sample relative to a non-cancerous biological sample, comprising;
mapping an isolated nucleic acid comprising at least one promoter sequence obtained from said cancerous biological sample against a reference nucleic acid obtained from said non-cancerous biological sample to obtain a read per kilo-base per million (RPKM) value or fragments per kilo-base per million (FPKM) value for said at least one promoter; and
determining the differential activity of the at least one promoter sequence in the nucleic acid relative to the activity of the at least one promoter in the reference nucleic acid sequence using said RPKM or FPKM value wherein the step of determining the differential activity of the at least one promoter sequence comprises determining that the RKPM or FPKM value for the at least one promoter in the nucleic acid obtained from the cancerous biological sample is:
(i) greater than between a 1 to 20-fold, such as a 1-fold, 2-fold, 3-fold, 4-fold or 5-fold, change in mean RPKM or FPKM value relative to the RPKM or FPKM value of the at least one promoter in the reference nucleic acid obtained from the non-cancerous biological sample; and
(ii) greater than a 0.1 RPKM or FPKM range relative to the RPKM or FPKM value of the at least one promoter in the reference nucleic acid obtained from the non-cancerous biological sample;
wherein the cancer is gastric cancer; and
wherein the nucleic acid is isolated from said cancerous biological sample by immunoprecipitation of chromatin, wherein said nucleic acid comprises said at least one promoter, the immunoprecipitation being achieved by an antibody specific for a modified histone protein comprising at least one histone modification selected from the group consisting of H3K4me3, H3K4me1, and H3K27ac.

2. The method of claim 1, wherein the cancerous and non-cancerous biological sample comprises a single cell, multiple cells, fragments of cells, body fluid or tissue, particularly wherein the cancerous and non-cancerous biological sample is obtained from the same subject, or wherein the cancerous and non-cancerous biological sample are each obtained from different subjects.

3. The method of any one of claims 1-2, wherein the antibody is specific to the at least one histone modification selected from the group consisting of H3K4me3, H3K4me1 and H3K27ac.

4. The method of any one of claims 1-3, wherein the isolated nucleic acid comprising at least one promoter is amplified with at least one primer, particularly wherein said amplified nucleic acid is used to construct a nucleic acid sequence library with said amplified nucleic acid.

5. The method of any one of claims 1-4, wherein the mapping step comprises calculating the RPKM values based upon the total sequence tags for the at least one promoter in the mapped nucleic acid relative to the reference nucleic acid, or wherein the mapping step comprises calculating the FPKM values based upon identified transcript sequences associated with the at least one promoter in the mapped nucleic acid relative to the reference nucleic acid.

6. The method of any one of claims 1-5, wherein the at least one promoter comprises an increase of SUZ12 binding sites relative to the total promoter population, and/or wherein the at least one promoter comprises a cryptic promoter.

7. The method of any one of claims 1-6, wherein the at least one promoter is positioned adjacent to a gene associated with cell-type specification, embryonic development or transcription factors, particularly wherein the at least one promoter is positioned adjacent to a gene associated with cancer the gene preferably being NKX6-3, SALL4, HOXB9, MET, TNK2, KLK1, FAR2, HOXA11 or HOXA11-AS.

8. The method of any one of claims 1-7, further comprising:
generating a matrix of sequencing tag counts for said at least one promoter based on the mapping step; and
analyzing said matrix of sequencing tag counts prior to determining the differential activity the at least one promoter sequence in the nucleic acid.

9. The method of claim 8, wherein the generating step comprises calculating the matrix based upon a sequence tag count for the at least one promoter in the mapped nucleic acid relative to the reference nucleic acid, or wherein the analysis step comprises analyzing the matrix using a DESeq2 algorithm, or wherein the differential enrichment is identified based upon an FDR rate of 10% and an absolute fold change of greater than 1.5.

10. The method of claim 8, wherein the at least one promoter is positioned adjacent to a gene associated with cancer, the gene preferably being RASA3, GRIN2D, TNNI3, SHD, ATP10B, SMTN, MYO15B, C2orf61, LINC00443 or ACHE.

## Patentansprüche

1. Verfahren zum Bestimmen der Gegenwart oder Aktivität von zumindest einem Promotor in einer kanzerösen biologischen Probe relativ zu einer nicht-kanzerösen biologischen Probe, welches umfasst:
Kartieren einer isolierten Nukleinsäure, die zumindest eine Promotorsequenz aufweist, und die aus der kanzerösen biologischen Probe erhalten wurde, gegen eine Referenznukleinsäure, die aus der nicht-kanzerösen biologischen Probe erhalten wurde, um einen " read per kilo-base per million" (RPKM) Wert oder einen "fragments per kilo-base per million" (FPKM) Wert für den zumindest einen Promotor zu erhalten; und
Bestimmen der differentiellen Aktivität der zumindest einen Promotorsequenz in der Nukleinsäure relativ zu der Aktivität des zumindest einen Promotors in der Referenznukleinsäuresequenz unter Verwendung des RPKM oder FPKM Werts, wobei der Schritt des Bestimmens der differentiellen Aktivität der zumindest einen Promotorsequenz die Bestimmung umfasst, dass der RPKM oder FPKM Wert für den zumindest einen Promotor in der Nukleinsäure, die aus der kanzerösen biologischen Probe erhalten wurde:
(i) größer ist als zwischen einer 1- bis 20-fachen, wie etwa einer 1-fachen, 2-fachen, 3-fachen, 4-fachen oder 5-fachen, Änderung des mittleren RPKM oder FPKM Werts relativ zu dem RPKM oder FPKM Wert des zumindest einen Promotors in der Referenznukleinsäure, die aus der nicht-kanzerösen biologischen Probe erhalten wurde; und
(ii) größer ist als eine 0.1-fache RPKM oder FPKM Größenordnung relativ zu dem RPKM oder FPKM Wert des zumindest einen Promotors in der Referenznukleinsäure, die aus der nicht-kanzerösen biologischen Probe erhalten wurde;
wobei der Krebs Darmkrebs ist; und
wobei die Nukleinsäure aus der kanzerösen biologischen Probe durch Immunpräzipitation von Chromatin isoliert wurde, wobei die Nukleinsäure zumindest einen Promotor aufweist, und wobei die Immunpräzipitation mit einem Antikörper bewerkstelligt wurde, der spezifisch für ein modifiziertes Histon-Protein ist, das zumindest eine Histon-Modifikation aufweist, welche aus der Gruppe ausgewählt ist, die aus H3K4me3, H3K4me1 und H3K27ac besteht.

2. Verfahren gemäß Anspruch 1, wobei die kanzeröse und die nicht-kanzeröse biologische Probe eine einzelne Zelle, multiple Zellen, Fragmente von Zellen, Körperflüssigkeit oder -gewebe umfasst, und insbesondere wobei die kanzeröse und die nicht-kanzeröse biologische Probe aus dem selben Subjekt erhalten werden, oder wobei die kanzeröse und die nicht-kanzeröse biologische Probe jeweils aus verschiedenen Subjekten erhalten werden.

3. Verfahren gemäß einem der Ansprüche 1 oder 2, wobei der Antikörper spezifisch für die zumindest eine Histon-Modifikation ist, welche aus der Gruppe ausgewählt ist, die aus H3K4me3, H3K4me1 und H3K27ac besteht.

4. Verfahren gemäß einem der Ansprüche 1-3, wobei die isolierte Nukleinsäure, die zumindest einen Promotor aufweist, mit zumindest einem Primer amplifiziert wird, und insbesondere wobei die amplifizierte Nukleinsäure verwendet wird, um eine Nukleinsäuresequenzbibliothek mit der amplifizierten Nukleinsäure zu konstruieren.

5. Verfahren gemäß einem der Ansprüche 1-4, wobei der Schritt des Kartierens das Berechnen der RPKM Werte basierend auf den gesamten Sequenz-Tags für den zumindest einen Promotor in der kartierten Nukleinsäure relativ zu der Referenznukleinsäure umfasst, oder wobei der Schritt des Kartierens das Berechnen der FPKM Werte basierend auf den identifizierten Transkriptsequenzen umfasst, welche mit dem zumindest einen Promotor in der kartierten Nukleinsäure relativ zu der Referenznukleinsäure assoziiert sind.

6. Verfahren gemäß einem der Ansprüche 1-5, wobei der zumindest eine Promotor eine Erhöhung an SUZ12 Bindungsstellen relativ zu der gesamten Promotorpopulation aufweist, und/oder wobei der zumindest eine Promotor einen kryptischen Promotor aufweist.

7. Verfahren gemäß einem der Ansprüche 1-6, wobei der zumindest eine Promotor angrenzend zu einem Gen positioniert ist, das mit Zelltypspezifikation, Embryonalentwicklung oder Transkriptionsfaktoren assoziiert ist, und insbesondere wobei der zumindest eine Promotor angrenzend zu einem Gen positioniert ist, das mit Krebs assoziiert ist, und wobei das Gen vorzugsweise NKX6-3, SALL4, HOXB9, MET, TNK2, KLK1, FAR2, HOXA11 oder HOXA11-AS ist.

8. Verfahren gemäß einem der Ansprüche 1-7, das ferner umfasst:
Generieren einer Matrix aus Zählungen von Sequenzierungs-Tags für den zumindest einen Promotor basierend auf dem Schritt des Kartierens; und
Analysieren der Matrix aus Zählungen von Sequenzierungs-Tags vor dem Bestimmen der differentiellen Aktivität der zumindest einen Promotorsequenz in der Nukleinsäure.

9. Verfahren gemäß Anspruch 8, wobei der Schritt des Generierens das Berechnen der Matrix basierend auf einer Zählung von Sequenz-Tags für den zumindest einen Promotor in der kartierten Nukleinsäure relativ zu der Referenznukleinsäure umfasst, oder wobei der Schritt des Analysierens das Analysieren der Matrix unter Verwendung eines DESeq2 Algorithmus umfasst, oder wobei die differentielle Anreicherung basierend auf einer FDR Rate von 10% und einer absoluten Änderung von mehr als 1.5-fach identifiziert wird.

10. Verfahren gemäß Anspruch 8, wobei der zumindest eine Promotor angrenzend zu einem Gen positioniert ist, das mit Krebs assoziiert ist, und wobei das Gen vorzugsweise RASA3, GRIN2D, TNNI3, SHD, ATP10B, SMTN, MYO15B, C2orf61, LINC00443 oder ACHE ist.

## Revendications

1. Procédé pour déterminer la présence ou l'activité d'au moins un promoteur dans un échantillon biologique cancéreux par rapport à un échantillon biologique non cancéreux, comprenant:
mappage d'un acide nucléique isolé comprenant au moins une séquence promotrice obtenue à partir dudit échantillon biologique cancéreux avec un acide nucléique de référence obtenu à partir dudit échantillon biologique non cancéreux pour obtenir une valeur de "read per kilo-base per million" (RPKM) ou des "fragments per kilo-base per million" (FPKM) pour ledit au moins un promoteur; et
déterminer de l'activité différentielle d'au moins une séquence promotrice dans l'acide nucléique par rapport à l'activité d'au moins un promoteur dans la séquence d'acide nucléique de référence en utilisant ladite valeur RPKM ou FPKM, l'étape de détermination de l'activité différentielle de l'au moins un La séquence promotrice comprend la détermination que la valeur de RKPM ou de FPKM pour le ou les promoteurs de l'acide nucléique obtenu à partir de l'échantillon biologique cancéreux est:
(i) supérieur à 1 à 20 fois, tel qu'un changement de 1 fois, 2 fois, 3 fois, 4 fois ou 5 fois, de la valeur moyenne de RPKM ou de FPKM par rapport à la valeur de RPKM ou de FPKM du ou des promoteurs dans l'acide nucléique de référence obtenu à partir de l'échantillon biologique non cancéreux; et
(ii) supérieur à une plage de 0,1 RPKM ou FPKM par rapport à la valeur RPKM ou FPKM de l'au moins un promoteur dans l'acide nucléique de référence obtenu à partir de l'échantillon biologique non cancéreux;
dans lequel le cancer est un cancer gastrique; et
dans lequel l'acide nucléique est isolé dudit échantillon biologique cancéreux par immunoprécipitation de la chromatine, dans lequel ledit acide nucléique comprend ledit au moins un promoteur, l'immunoprécipitation étant obtenue par un anticorps spécifique d'une protéine histone modifiée comprenant au moins une modification d'histone sélectionnée dans le groupe constitué de H3K4me3, H3K4me1 et H3K27ac.

2. Procédé selon la revendication 1, dans lequel l'échantillon biologique cancéreux et non cancéreux comprend une cellule unique, plusieurs cellules, fragments de cellules, fluide corporel ou tissu, en particulier dans lequel l'échantillon biologique cancéreux et non cancéreux est obtenu du même sujet, ou dans lesquels les échantillons biologiques cancéreux et non cancéreux sont chacun obtenus de différents sujets.

3. Procédé selon l'une quelconque des revendications 1 à 2, dans lequel l'anticorps est spécifique de la au moins une modification d'histone choisie dans le groupe constitué de H3K4me3, H3K4me1 et H3K27ac.

4. Procédé selon l'une quelconque des revendications 1 à 3, dans lequel l'acide nucléique isolé comprenant au moins un promoteur est amplifié avec au moins une amorce, en particulier dans lequel ledit acide nucléique amplifié est utilisé pour construire une bibliothèque de séquences d'acide nucléique avec ledit acide nucléique amplifié.

5. Procédé selon l'une quelconque des revendications 1 à 4, dans lequel l'étape de cartographie comprend le calcul des valeurs de RPKM sur la base des étiquettes de séquence totales pour le au moins un promoteur dans l'acide nucléique mappé par rapport à l'acide nucléique de référence, ou dans lequel l'étape de cartographie comprend calculer les valeurs de FPKM sur la base de séquences de transcription identifiées associées au moins un promoteur dans l'acide nucléique cartographié par rapport à l'acide nucléique de référence.

6. Procédé selon l'une quelconque des revendications 1 à 5, dans lequel le au moins un promoteur comprend une augmentation des sites de liaison de SUZ12 par rapport à la population totale de promoteurs, et/ou dans lequel le au moins un promoteur comprend un promoteur cryptique.

7. Procédé selon l'une quelconque des revendications 1 à 6, dans lequel le au moins un promoteur est positionné de manière adjacente à un gène associé à des facteurs de spécification de type cellulaire, de développement embryonnaire ou de transcription, en particulier dans lequel le au moins un promoteur est positionné de manière adjacente à un gène associé. avec le cancer, le gène est de préférence NKX6-3, SALL4, HOXB9, MET, TNK2, KLK1, FAR2, HOXA11 ou HOXA11-AS.

8. Procédé selon l'une quelconque des revendications 1 à 7, comprenant en outre:
générer une matrice de comptage d'étiquettes de séquençage pour ledit au moins un promoteur sur la base de l'étape de mappage; et
analyser ladite matrice de comptage de marqueurs de séquençage avant de déterminer l'activité différentielle d'au moins une séquence promotrice dans l'acide nucléique.

9. Procédé selon la revendication 8, dans lequel l'étape de génération comprend le calcul de la matrice sur la base d'un nombre d'étiquettes de séquence pour le au moins un promoteur dans l'acide nucléique mappé par rapport à l'acide nucléique de référence, ou dans lequel l'étape d'analyse comprend l'analyse de la matrice en utilisant un DESeq2 algorithme, ou dans lequel l'enrichissement différentiel est identifié sur la base d'un taux de FDR de 10% et d'un changement de pli absolu supérieur à 1.5.

10. Procédé selon la revendication 8, dans lequel le au moins un promoteur est positionné adjacent à un gène associé au cancer, le gène étant de préférence RASA3, GRIN2D, TNNI3, SHD, ATP10B, SMTN, MYO15B, C2orf61, LINC00443 ou ACHE.
